(19) Europäisches Patentamt
European Patent Office
Office européen des brevets

(11) EP 1 586 307 A2

(12) # EUROPÄISCHE PATENTANMELDUNG

(43) Veröffentlichungstag:
**19.10.2005 Patentblatt 2005/42**

(51) Int Cl.[7]: **A61K 7/48**

(21) Anmeldenummer: **05101289.6**

(22) Anmeldetag: **21.02.2005**

(84) Benannte Vertragsstaaten:
**AT BE BG CH CY CZ DE DK EE ES FI FR GB GR HU IE IS IT LI LT LU MC NL PL PT RO SE SI SK TR**
Benannte Erstreckungsstaaten:
**AL BA HR LV MK YU**

(30) Priorität: **23.03.2004 DE 102004014616**

(71) Anmelder: **Beiersdorf AG**
**20245 Hamburg (DE)**

(72) Erfinder:
- **Biergiesser, Helga**
  **21465, Reinbek (DE)**
- **Von der Fecht, Stephanie**
  **22869, Schenefeld (DE)**
- **Schulz, Jens**
  **22869, Schenefeld (DE)**
- **Eckert, Julia**
  **20257, Hamburg (DE)**
- **Raschke, Thomas**
  **25421, Pinneberg (DE)**

(54) **Kosmetische und dermatologische Zubereitungen mit konserviertem Taurin**

(57) Kosmetische oder dermatologische Zubereitungen enthaltend Taurin und mindestens einen Stoff gewählt aus der Gruppe Parabene, Sorbinsäure oder deren Alkalisalze, oder IPBC.

EP 1 586 307 A2

## Beschreibung

Die vorliegende Erfindung betrifft kosmetische und dermatologische Zubereitungen, insbesondere hautpflegende kosmetische und dermatologische Emulsionen mit einem Gehalt an Taurin.
Die Haut ist das größte Organ des Menschen. Unter ihren vielen Funktionen (beispielsweise zur Wärmeregulation und als Sinnesorgan) ist die Barrierefunktion, die das Austrocknen der Haut (und damit letzlich des gesamten Organismus) verhindert, die wohl wichtigste. Gleichzeitig wirkt die Haut als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Die Epidermis ist ein stratifiziertes Gewebe, in dem die äußere Schicht, die Hornschicht (Stratum corneum), den für die Barrierefunktion bedeutenden Teil darstellt. Das heute in der Fachwelt anerkannte Hautmodell von Elias (P. M. Elias, Structure and Function of the Stratum Corneum Permeability Barrier, Drug Dev. Res. 13, 1988, 97-105) beschreibt die Hornschicht als Zwei-Komponenten-System, ähnlich einer Ziegelsteinmauer (Ziegelstein-Mörtel-Modell). In diesem Modell entsprechen die Hornzellen (Korneozyten) den Ziegelsteinen, die komplex zusammengesetzte Lipidmembran in den Interzellularräumen entspricht dem Mörtel. Dieses System stellt im wesentlichen eine physikalische Barriere gegen hydrophile Substanzen dar, kann aber aufgrund seiner engen und mehrschichtigen Struktur gleichermaßen auch von lipophilen Substanzen nur schwer passiert werden.

Die vorliegende Erfindung betrifft in einer besonderen Ausführungsform kosmetische oder pharmazeutische Zubereitungen mit vermindertem Klebrigkeitsgefühl, Verfahren zu ihrer Herstellung sowie die Verwendung von Wirkstoffen zur Herabminderung des Klebrigkeitsgefühles kosmetischer Zubereitungen.

Außer ihrer Barrierewirkung gegen externe chemische und physikalische Einflüsse tragen die epidermalen Lipide auch zum Zusammenhalt der Hornschicht bei und haben Einfluß auf die Hautglätte. Im Gegensatz zu den Talgdrüsenlipiden, die keinen geschlossenen Film auf der Haut ausbilden, sind die epidermalen Lipide über die gesamte Hornschicht verteilt.

Das äußerst komplexe Zusammenwirken der feuchtigkeitsbindenden Substanzen und der Lipide der oberen Hautschichten ist für die Regulation der Hautfeuchte sehr wichtig. Daher enthalten Kosmetika in der Regel, neben ausgewogenen Lipidabmischungen und Wasser, wasserbindende Substanzen.

Neben der chemischen Zusammensetzung ist jedoch auch das physikalische Verhalten dieser Substanzen von Bedeutung. Daher ist die Entwicklung von sehr gut bioverträglichen Emulgatoren bzw. Tensiden wünschenswert. Damit formulierte Produkte unterstützen die flüssigkristalline Organisation der Interzellularlipide des Stratum Corneums und verbessern so die Barriereeigenschaften der Hornschicht. Besonders vorteilhaft ist es, wenn deren Molekülbestandteile aus natürlicherweise in der Epidermis vorkommenden Substanzen bestehen.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Übliche kosmetische Darreichungsformen sind Emulsionen. Darunter versteht man im allgemeinen ein heterogenes System aus zwei miteinander nicht oder nur begrenzt mischbaren Flüssigkeiten, die üblicherweise als Phasen bezeichnet werden. Die eine liegt dabei in Form von Tröpfchen vor (dispere oder innere Phase), während die andere Flüssigkeit eine kontinuierliche (kohärente oder innere Phase) bildet. Seltenere Darreichungsformen sind multiple Emulsionen, also solche, welche in den Tröpfchen der dispergierten (oder diskontinuierlichen) Phase ihrerseits Tröpfchen einer weiteren dispergierten Phase enthalten, z.B. W/O/W-Emulsionen und O/W/O-Emulsionen.

Liegt die Ölphase fein verteilt in der Wasserphase vor, so handelt es sich um eine Ölin-Wasser-Emulsion (O/W-Emulsion, z. B. Milch). Der Grundcharakter einer O/W-Emulsion ist durch das Wasser geprägt, d. h. sie wirkt im allgemeinen weniger fettend auf der Haut, ist eher mattierend und zieht schneller in die Haut ein als eine W/O-Emulsion.

Natürlich ist dem Fachmann eine Vielzahl von Möglichkeiten bekannt, stabile O/W - Zubereitungen zur kosmetischen oder dermatologischen Anwendung zu formulieren, beispielsweise in Form von Cremes und Salben, die im Bereich von Raum- bis Hauttemperatur streichfähig sind, oder als Lotionen und Milche, die in diesem Temperaturbereich eher fließfähig sind.

Die Stabilität von Emulsionen ist u. a. von ihrer Viskosität, insbesondere von der Viskosität der äußeren Phase abhängig. Eine Emulsion wird dann instabil, wenn sich die feindispergierten Teilchen wieder zu größeren Aggregaten zusammenballen und die sich berührenden Tröpfchen zusammenfließen. Dieser Vorgang wird als Koaleszenz bezeichnet. Der Prozeß der Koaleszenz läuft umso langsamer ab, je viskoser die äu-ßere Phase der Emulsion ist.

Emulsionen von "flüssiger" (= fließfähiger) Konsistenz finden in der Kosmetik beispielsweise als Pflege-, Reinigungs-, Gesichts- oder Handlotion Verwendung. Sie haben in der Regel eine Viskosität von etwa 2000 mPa·s bis zu etwa 10 000 mPa·s. Der Stabilität von fließfähigen Emulsionen ist besondere Aufmerksamkeit zu widmen, da die erheblich größere Beweglichkeit der Teilchen eine schnellere Koaleszenz fördert.

Auch flüssige Emulsionen des Standes der Technik sind - da auch sie i. a. Verdickungsmittel enthalten - gegenüber höheren Elektrolytkonzentrationen nicht stabil, was sich in einer Phasentrennung äußert. Es ist aber häufig wünschenswert, bestimmte Elektrolyte, wie beispielsweise wasserlösliche UV-Filter, einzusetzen, um deren sonstige physikalische, chemische bzw. physiologische Eigenschaften nutzen zu können. Zwar läßt sich in vielen Fällen durch geeignete Wahl des Emulgatorsystems in gewissem Maß Abhilfe schaffen, es treten dann aber ebensooft andere Nachteile auf.

Die angesprochenen Nachteile können beispielsweise darin liegen, daß Emulgatoren, wie letztendlich jede chemische Substanz, im Einzelfall allergische oder auf Überempfindlichkeit des Anwenders beruhende Reaktionen hervorrufen können, obwohl die Verwendung der üblichen kosmetischen Emulgatoren i. a. natürlich völlig unbedenklich ist.

Um die Metastabilität von Emulsionen gewährleisten zu können, sind in der Regel grenzflächenaktive Substanzen, also Emulgatoren, nötig. An sich ist die Verwendung der üblichen kosmetischen Emulgatoren völlig unbedenklich. Dennoch können Emulgatoren, wie letztlich jede chemische Substanz, im Einzelfalle allergische oder auf Überempfindlichkeit des Anwenders beruhende Reaktionen hervorrufen. So ist bekannt, daß bei manchen besonders empfindlichen Personen bestimmte Lichtdermatosen durch gewisse Emulgatoren und gleichzeitige Einwirkung von Sonnenlicht ausgelöst werden.

Es ist möglich, emulgatorfreie Zubereitungen herzustellen, welche beispielsweise in einer Ölphase dispergierte Wassertröpfchen, beziehungsweise in einer Wasserphase dispergierte Öltröpfchen, aufweisen. Solche Systeme werden gelegentlich Oleodispersionen oder Hydrodispersionen genannt, je nachdem, welches die disperse und welches die kontinuierliche Phase darstellt.

Es ist für die kosmetische Galenik aber weder nötig noch möglich, auf Emulgatoren ganz zu verzichten, zumal eine gewisse Auswahl an besonders milden Emulgatoren existiert. Allerdings besteht ein Mangel des Standes der Technik an einer befriedigend großen Vielfalt solcher Emulgatoren, welche dann auch das Anwendungsspektrum entsprechend milder und hautverträglicher kosmetischer Zubereitungen deutlich verbreitern würde.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubsubstanzen zur Verfügung zu haben, da auch dessen Strahlen Schäden hervorrufen können. Man hat lange Zeit fälschlicherweise angenommen, daß die langwellige UV-A-Strahlung mit einer Wellenlänge zwischen 320 nm und 400 nm nur eine vernachlässigbare biologische Wirkung aufweist und daß dementsprechend die UV-B-Strahlen für die meisten Lichtschäden an der menschlichen Haut verantwortlich seien. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

So ist es u.a. erwiesen, daß selbst die UV-A-Strahlung unter ganz normalen Alltagsbedingungen ausreicht, um innerhalb kurzer Zeit die Collagen- und Elastinfasern zu schädigen, die für die Struktur und Festigkeit der Haut von wesentlicher Bedeutung sind. Hierdurch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung

aus.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z.B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.

Vorbeugender Schutz gegen UV-A-Strahlen, beispielsweise durch Auftrag von Lichtschutzfiltersubstanzen in Form einer kosmetischen oder dermatologischen Formulierung auf die Haut, ist daher von grundsätzlicher Wichtigkeit.

Im allgemeinen ist das Lichtabsorptionsverhalten von Lichtschutzfiltersubstanzen sehr gut bekannt und dokumentiert, zumal in den meisten Industrieländern Positivlisten für den Einsatz solcher Substanzen existieren, welche recht strenge Maßstäbe an die Dokumentation anlegen. Für die Dosierung der Substanzen in den fertigen Formulierungen können die Extinktionswerte allenfalls eine Orientierungshilfe bieten, denn durch Wechselwirkungen mit Inhaltsstoffen der Haut oder der Hautoberfläche selbst können Unwägbarkeiten auftreten. Ferner ist in der Regel schwierig vorab abzuschätzen, wie gleichmäßig und in welcher Schichtdicke die Filtersubstanz in und auf der Hornschicht der Haut verteilt ist.

Zur Prüfung der UV-A-Schutzleistung wird üblicherweise die IPD-Methode verwendet (IPD = immediate pigment darkening). Hierbei wird - ähnlich der Bestimmung des Lichtschutzfaktors - ein Wert ermittelt, der angibt, um wieviel länger die mit dem Lichtschutzmittel geschützte Haut mit UV-A-Strahlung bestrahlt werden kann, bis die gleiche Pigmentierung auftritt wie bei der ungeschützten Haut.

Eine andere, europaweit etablierte Prüfungsmethode ist der Australische Standard AS/NZS 2604:1997. Dabei wird die Absorption der Zubereitung im UV-A-Bereich gemessen. Um den Standard zu erfüllen, muß die Zubereitung mindestens 90 % der UV-A-Strahlung im Bereich 320 -360 nm absorbieren.

Die Einsatzkonzentration bekannter Lichtschutzfiltersubstanzen, die insbesondere auch im UV-A-Bereich eine hohe Filterwirkung zeigen, ist häufig gerade in Kombination mit anderen als Festkörper vorliegenden Substanzen begrenzt. Es bereitet daher gewisse formulierungstechnische Schwierigkeiten, höhere Lichtschutzfaktoren bzw. UV-A-Schutzleistung zu erzielen.

Da Lichtschutzfiltersubstanzen in der Regel kostspielig sind und da manche Lichtschutzfiltersubstanzen zudem schwierig in höheren Konzentrationen in kosmetische oder dermatologische Zubereitungen einzuarbeiten sind, war es eine Aufgabe der Erfindung, auf einfache und preiswerte Weise zu Zubereitungen zu gelangen, welche bei ungewöhnlich niedrigen Konzentrationen an herkömmlichen UV-A-Lichtschutzfiltersubstanzen dennoch eine akzeptable oder sogar hohe UV-A-Schutzleistung erreichen.

Die UV-Strahlung kann aber auch zu photochemischen Reaktionen führen, wobei dann die photochemischen Reaktionsprodukte in den Hautmetabolismus eingreifen. Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Tripiettsauerstoff (radikalischer Grundzustand) durch gesteigerte Reaktivität aus. Allerdings existieren auch angeregte, reaktive (radikalische) Triplettzustände des Sauerstoffmoleküls.

Um diesen Reaktionen vorzubeugen, können den kosmetischen bzw. dermatologischen Formulierungen zusätzlich Antioxidantien und/oder Radikalfänger einverleibt werden.

Die Verbindungen, welche als Lichtschutzmittel für kosmetische und dermatologische Lichtschutzformulierungen eingesetzt werden, zeichnen sich meist an sich durch gute Lichtschutzwirkung aus. Sie haben jedoch den Nachteil, daß es bisweilen schwierig ist, sie in befriedigender Weise solchen Formulierungen einzuverleiben.

Der Lichtschutzfaktor (LSF, oft auch, dem englischen Sprachgebrauch angepaßt, SPF genannt) gibt an, um wieviel länger die mit dem Lichtschutzmittel geschützte Haut bestrahlt werden kann, bis die gleiche Erythemreaktion auftritt wie bei der ungeschützten Haut (also zehnmal solang gegenüber ungeschützter Haut bei LSF = 10).

Jedenfalls erwartet der Verbraucher zum einen - nicht zuletzt wegen der ins Licht der Öffentlichkeit gerückten Diskussion über das sogenannte "Ozonloch" zum einen zuverlässige Angaben des Herstellers zum Lichtschutzfaktor, zum anderen geht eine Tendenz des Verbrauchers zu höheren und hohen Lichtschutzfaktoren.

Da Lichtschutzfiltersubstanzen in der Regel kostspielig sind, und da manche Lichtschutzfiltersubstanzen zudem schwierig in höheren Konzentrationen in kosmetische oder dermatologische Zubereitungen einzuarbeiten sind, war es eine weitere Aufgabe der Erfindung, auf einfache und preiswerte Weise zu Zubereitungen zu gelangen, welche selbst bei ungewöhnlich niedrigen Konzentrationen an Lichtschutzfiltersubstanzen dennoch akzeptable oder sogar hohe LSF-Werte erreichen.

Ferner war es eine Aufgabe der vorliegenden Erfindung, kosmetische oder dermatologische Lichtschutzzubereitungen zu konzipieren, welche sich durch erhöhte Pflegewirkung auszeichnen.

Die Austrocknung der Haut führt zu einem Anstieg der Osmolarität in den äußeren Schichten der Epidermis. Die Erhöhung der Osmolarität bedingt insbesondere infolge der gesteigerten Konzentration an NaCl eine Hypertonizität mit nachfolgender Zellschrumpfung. Taurin, als hauteigenes Aminosäurederivat wird von den Keratinozyten aktiv aufgenommen (Janeke et al., J. Invest. Dermatol. 121: 354-361, 2003) und stabilisiert als organischer Osmolyt das Zellvolumen und somit die davon abhängigen Vorgänge des Zellstoffwechsels unter hypotonischen Bedingungen.

Im menschlichen Körper ist Taurin das biologische Amin der Cysteinsäure, das beim Abbau der Aminosäure "Cystein" im Körper gebildet wird. BDF bezieht Taurin (EI-NECS 2034838; CAS 107-35-7; Chemical name 2- Aminoethanesulfonic acid, Strukturformel$C_2H_7NSO_3$), welches unter der internationalen Bezeichnung Taurin/96123, von der Firma Ajinomoto.

Taurin wird zur in Konzentrationsbereichen von 0.1-3% eingesetzt, besonders bevorzugt sind Einsatzbereiche zwischen 0,2- 0,5%, am besten 0,3%.

Aufrgund seiner Wasserlöslichkeit wird es in verschiedenen Grundlagen wie O/W-Cremes, W/O-Cremes, PIT-Emulsionen, Hydrodispersionen usw eingesetzt. Taurin weist bei unterschiedlichen pH-Werten (5<pH<7) unter den üblichen Lagerbedingungen (+6°C, -10°C, Schaukeltest) eine sehr gute Stabilität auf.

Taurin ist al schwefelhaltiges Aminosäure-Analogon insbesondere gegenüber gramnegativen Bakterien instabil, vermutlich weil einige dieser Bakterien Schwefelverbindungen verstoffwechseln. Daher stellt Taurin für diese Bakterien einen geeigneten Nährboden dar, was bedeuet, das Taurin enthaltende Zubereitungen eine mikrobielle Instabilität aufweisen.

Es war indes überraschend und für den Fachmann nicht vorauszusehen, daß kosmetische oder dermatologische Zubereitungen enthaltend Taurin und einen Stoff gewählt aus der Gruppe Parabene, Sorbinsäure oder deren Alkalisalze, IPBC den Nachteilen des Standes der Technik abhelfen.

Parabene sind p-Hydroxybenzoesäureester, insbesondere der Methyl-, Ethyl-, Propyl-, iso-Propyl-, Phenoxyethyl-, Benzyl-, n-Butylester, besonders bevorzugt der Methyl- oder Propylester.

IPBC ist (3-Iod-2-propinyl)-N-butylcarbamat, CAS-Nr. 55406-53-6. Es wird unter anderem als Zusatzstoff für Anstrichmittel verwendet.

Parabene, Sorbinsäure und IPBC werden üblicherweise gegen grampositive und nicht gegen gramnegative Bakterien eingesetzt. Es war für den Fachmann ferner nicht vorauszusehen gewesen, daß die erfindungsgemäßen Zubereitungen

- besser als feuchtigkeitsspendende Zubereitungen wirken,
- besser die Hautglättung fördern,
- sich durch bessere Pflegewirkung auszeichnen,
- höhere Stabilität gegenüber der Kristallisation der eingesetzten Rohstoffe aufweisen
- sich durch bessere Bioverträglichkeit auszeichnen würden
- sich durch ein besseres Hautgefühl und durch höhere kosmetische Eleganz auszeichnen würden
- sich durch eine bessere Behandlung und Ausbalancierung des Glucosaminoglykan-/Proteoglykanstoffwechsels
- sich durch einen bessere Ausgleich von Feuchtigkeitsdefiziten in oberen und tieferen Hautschichten (epidermal und dermal),
- sich zur strukturellen Verbesserung der Haut, insbesondere im Alter,
- sich zur besseren Verstärkung der epidermal-dermalen Junktionszone
- sich zur besseren Pflege und Prophylaxe lichtgealterter und/oder alterstrockener Haut,
- sich zur Ausbalancierung degenerativer Prozesse in allen Hautschichten
- sich zur Verbesserung der Kommunikation zwischen den einzelnen Hautschichten (dermal-epidermal crosstalk).
- sich besser zur Steigerung der Ankerfibrillen (Kollagen-7) und der Kollagensynthese und
- sich über eine breite kosmetische Variabilität auszeichnen würden und über breite Konsistenz- und Viskositätsbereiche von 400 mPas bis > 20.000 mPas formulieren lassen würden

als die Zubereitungen des Standes der Technik.

Weiter ist es bevorzugt, wenn der Stoff gewählt aus der Gruppe Parabene, Sorbinsäure, IPBC in Konzentrationen von 0.001 bis 1 Gew.%, besonders bevorzugt 0,01 bis 0,5 Gew.% vorliegt.

Weiter ist es bevorzugt, wenn Taurin in Konzentrationen von 0,001 bis 5 Gew.%, besonders bevorzugt 0.01-0.5 Gew.% vorliegt.

Weiter ist es bevorzugt, wenn das Verhältnis von Taurin zur Gesamtmenge an Stoffen gewählt aus der Gruppe Parabene, Sorbinsäure, IPBC 1:100 bis 1000:1, besonders bevorzugt 5:1 bis 1:5 beträgt.

Weiter ist es bevorzugt, wenn die Zubereitung in Form einer O/W-Emulsion oder Hydrodispersion vorliegt.

Die erfindungsgemäßen Zubereitungen sind besitzen sehr gute kosmetische Eigenschaften, insbesondere was die Klebrigkeit betrifft, und weisen eine sehr gute Hautverträglichkeit sowie Hautpflegeleistung auf.

Als Grundbestandteile der erfindungsgemäßen Zubereitungen können verwendet werden:

- Wasser oder wäßrige Lösungen
- wäßrige ethanolische Lösungen
- natürliche Öle und/oder chemisch modifizierte natürliche Öle und/oder synthetische Öle;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Octoxyglycerin, Ethylenglykol, Ethylenglykolmonoethyl- oder -monobutylether, Propylenglykolmonomethyl, -monoethyl- oder -monobutylether, Diethylenglykolmonomethyl- oder - monoethylether und analoge Produkte.

Insbesondere werden Gemische der vorstehend genannten Lösungsmittel verwendet.

Im Rahmen der vorliegenden Offenbarung wird als Oberbegriff für Fette, Öle, Wachse und dergleichen gelegentlich der Ausdruck "Lipide" verwendet, wie dem Fachmanne durchaus geläufig ist. Auch werden die Begriffe "Ölphase" und "Lipidphase" synonym angewandt.

Öle und Fette unterscheiden sich unter anderem in ihrer Polarität, welche schwierig zu definieren ist. Es wurde bereits vorgeschlagen, die Grenzflächenspannung gegenüber Wasser als Maß für den Polaritätsindex eines Öls bzw. einer Ölphase anzunehmen. Dabei gilt, daß die Polarität der betreffenden Ölphase umso größer ist, je niedriger die Grenzflächenspannung zwischen dieser Ölphase und Wasser ist. Erfindungsgemäß wird die Grenzflächenspannung als ein mögliches Maß für die Polarität einer gegebenen Ölkomponente angesehen.

Die Grenzflächenspannung ist diejenige Kraft, die an einer gedachten, in der Grenzfläche zwischen zwei Phasen befindlichen Linie der Länge von einem Meter wirkt. Die physikalische Einheit für diese Grenzflächenspannung errechnet sich klassisch nach der Beziehung Kraft/Länge und wird gewöhnlich in mN/m (Millinewton geteilt durch Meter) wiedergegeben. Sie hat positives Vorzeichen, wenn sie das Bestreben hat, die Grenzfläche zu verkleinern. Im umgekehrten Falle hat sie negatives Vorzeichen. Als polar im Sinne der vorliegenden Erfindung werden Lipide angesehen, deren Grenzflächenspannung gegen Wasser weniger als 30 mN/m beträgt.

Polare Öle, sind beispielsweise solche aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z.B. Olivenöl, Sonnenblumenöl, Sojaöl, Erdnußöl, Rapsöl, Ma n-delöl, Palmöl, Kokosöl, Rizinusöl, Weizenkeimöl, Traubenkernöl, Distelöl, Nachtkerzenöl, Macadamianußöl und dergleichen mehr.

Weitere polare Ölkomponenten können gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, wie z.B. Jojobaöl.

Ferner kann die Ölphase vorteilhaft gewählt werden aus der Gruppe der Dialkylether, der Gruppe der gesättigten oder ungesättigten, verzweigten oder unverzweigten Alkohole. Es ist insbesondere vorteilhaft, wenn die Ölphase der erfindungsgemäßen W/O-Emulsionen einen Gehalt an $C_{12-15}$-Alkylbenzoat aufweist oder vollständig aus diesem besteht.

Ferner kann die Ölphase vorteilhaft gewählt werden aus der Gruppe der Guerbetalkohole. Guerbetalkohole sind benannt nach Marcel Guerbet, der ihre Herstellung erstmalig beschrieb. Sie entstehen nach der Reaktionsgleichung

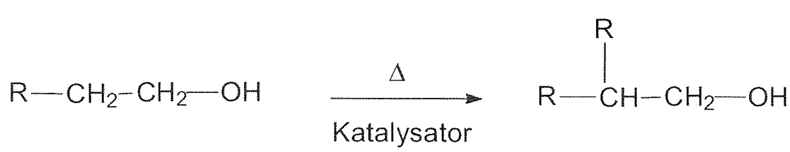


durch Oxidation eines Alkohols zu einem Aldehyd, durch Aldol-Kondensation des Aldehyds, Abspaltung von Wasser aus dem Aldol- und Hydrierung des Allylaldehyds. Guerbetalkohole sind selbst bei niederen Temperaturen flüssig und

bewirken praktisch keine Hautreizungen. Vorteilhaft können sie als fettende, überfettende und auch rückfettend wirkende Bestandteile in Haut- und Haarpflegemitteln eingesetzt werden.

Die Verwendung von Guerbet-Alkoholen in Kosmetika ist an sich bekannt. Solche Species zeichnen sich dann meistens durch die Struktur

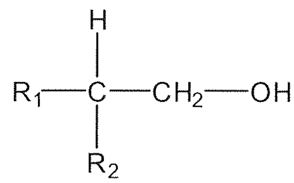

aus. Dabei bedeuten $R_1$ und $R_2$ in der Regel unverzweigte Alkylreste.

Erfindungsgemäß vorteilhaft werden der oder die Guerbet-Alkohole gewählt aus der Gruppe, bei denen
$R_1$ = Propyl, Butyl, Pentyl, Hexyl, Heptyl oder Octyl und
$R_2$ = Hexyl, Heptyl, Octyl, Nonyl, Decyl, Undecyl, Dodecyl, Tridecyl oder Tetradecyl.

Erfindungsgemäß bevorzugte Guerbet-Alkohole sind das 2-Butyloctanol - es hat die chemische Struktur

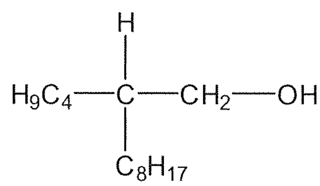

und ist beispielsweise unter der Handelsbezeichnung Isofol® 12 von der Gesellschaft Condea Chemie GmbH erhältlich - und das 2-Hexyldecanol - es hat die chemische Struktur

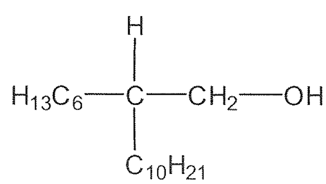

und ist beispielsweise unter der Handelsbezeichnung Isofol® 16 von der Gesellschaft Condea Chemie GmbH erhältlich.

Auch Mischungen von erfindungsgemäßen Guerbet-Alkoholen sind erfindungsgemäß vorteilhaft zu verwenden. Mischungen aus 2-Butyloctanol und 2-Hexyldecanol sind beispielsweise unter der Handelsbezeichnung Isofol® 14 von der Gesellschaft Condea Chemie GmbH erhältlich.

Die Gesamtmenge an Guerbet-Alkoholen in den fertigen kosmetischen oder dermatologischen Zubereitungen wird vorteilhaft aus dem Bereich bis 25,0 Gew.-%, bevorzugt 0,5 - 15,0 Gew.-% gewählt, bezogen auf das Gesamtgewicht der Zubereitungen.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Unpolare Öle sind beispielsweise solche, welche gewählt werden aus der Gruppe der verzweigten und unverzweigten Kohlenwasserstoffe und -wachse, insbesondere Vaseline (Petrolatum), Paraffinöl, Squalan und Squalen, Polyolefine und hydrogenierte Polyisobutene. Unter den Polyolefinen sind Polydecene die bevorzugten Substanzen. Die nachfolgende Tabelle 1 führt Lipide auf, die als Einzelsubstanzen oder auch im Gemisch untereinander erfindungsgemäß vorteilhaft sind. Die betreffenden Grenzflächenspannungen gegen Wasser sind in der letzten Spalte angegeben. Es ist jedoch auch vorteilhaft, Gemische aus höher- und niederpolaren und dergleichen zu verwenden.

Tabelle 1

| Handelsname | INCl-Bezeichnung | (mN/ m) |
|---|---|---|
| Isofol® 14 T | Butyl Decanol + Hexyl Decanol + Hexyl Octanol + Butyl Octanol | 27,6 |
| Isofof® 16 | Hexyl Decanol | 24,3 |
| Eutanol® G | Octyldodecanol | 24,8 |
| Cetiol® OE | Dicaprylyl Ether | 22,1 |
| Miglyol® 812 | Caprylic/Capric Triglyceride | 21,3 |
| Cegesoft® C24 | Octyl Palmitate | 23,1 |
| Isopropylstearat | Isopropyl Stearate | 21,9 |
| Estol® 1540 EHC | Octyl Octanoate | 30,0 |
| Finsolv® TN | $C_{12-15}$ Alkyl Benzoate | 21,8 |
| Cetiol® SN | Cetearyl Isonoanoate | 28,6 |
| Dermofeel® BGC | Butylene Glycol Dicaprylate/Dicaprate | 21,5 |
| Trivent® OCG | Tricaprylin | 20,2 |
| MOD | Octyldodeceyl Myristate | 22,1 |
| Cosmacol® ETI | Di-$C_{12-13}$ Alkyl Tartrate | 29,4 |
| Miglyol® 829 | Caprylic/Capric Diglyceryl Succinate | 29,5 |
| Prisorine® 2036 | Octyl Isostearate | 29,7 |
| Tegosoft® SH | Stearyl Heptanoate | 28,7 |
| Abil® Wax 9840 | Cetyl Dimethicone | 25,1 |
| Cetiol® LC | Coco-Caprylate/Caprate | 24,8 |
| IPP | Isopropyl Palmitate | 22,5 |
| Luvitol® EHO | Cetearyl Octanoate | 28,6 |
| Cetiol® 868 | Octyl Stearate | 28,4 |

Es kann ebenfalls vorteilhaft sein, die Ölphase der erfindungsgemäßen Zubereitungen teilweise oder vollständig aus der Gruppe der cyclischen und/oder linearen Silicone zu wählen, welche im Rahmen der vorliegenden Offenbarung auch als "Siliconöle" bezeichnet werden. Solche Silicone oder Siliconöle können als Monomere vorliegen, welche in der Regel durch Strukturelemente charakterisiert sind, wie folgt:

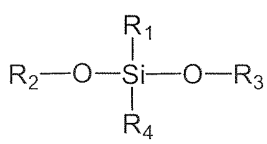

Als erfindungsgemäß vorteilhaft einzusetzenden linearen Silicone mit mehreren Siloxyleinheiten werden im allgemeinen durch Strukturelemente charakterisiert wie folgt:

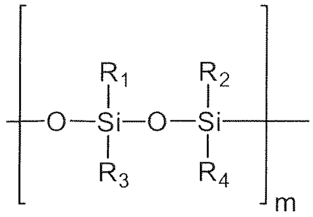
,

wobei die Siliciumatome mit gleichen oder unterschiedlichen Alkylresten und/oder Arylresten substituiert werden können, welche hier verallgemeinernd durch die Reste $R_1$ - $R_4$ dargestellt sind (will sagen, daß die Anzahl der unterschiedlichen Reste nicht notwendig auf bis zu 4 beschränkt ist). m kann dabei Werte von 2 - 200.000 annehmen.

Erfindungsgemäß vorteilhaft einzusetzende cyclische Silicone werden im allgemeinen durch Strukturelemente charakterisiert, wie folgt

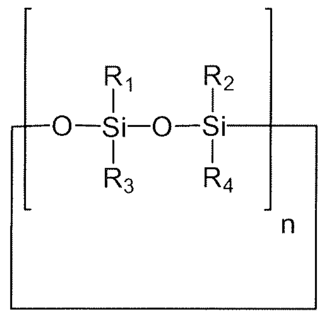

wobei die Siliciumatome mit gleichen oder unterschiedlichen Alkylresten und/oder Arylresten substituiert werden können, welche hier verallgemeinernd durch die Reste $R_1$ - $R_4$ dargestellt sind (will sagen, daß die Anzahl der unterschiedlichen Reste nicht notwendig auf bis zu 4 beschränkt ist). n kann dabei Werte von 3/2 bis 20 annehmen. Gebrochene Werte für n berücksichtigen, daß ungeradzahlige Anzahlen von Siloxylgruppen im Cyclus vorhanden sein können.

Vorteilhaft wird Phenyltrimethicon als Siliconöl gewählt. Auch andere Silikonöle, beispielsweise Dimethicon, Phenyldimethicon, Cyclomethicon (Octamethylcyclotetrasiloxan) beispielsweise Hexamethylcyclotrisiloxan, Polydimethylsiloxan, Poly(methylphenylsiloxan), Cetyldimethicon, Behenoxydimethicon sind vorteilhaft im Sinne der vorliegenden Erfindung zu verwenden.

Vorteilhaft sind ferner Mischungen aus Cyclomethicon und Isotridecylisononanoat, sowie solche aus Cyclomethicon und 2-Ethylhexylisostearat.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Polysiloxan-polyalkyl-polyether-copolymere wie das Cetyl-Dimethicon-Copolyol, das (Cetyl-Dimethicon-Copolyol (und) Polyglyceryl-4-Isostearat (und) Hexyllaurat)

Ferner kann die Ölphase vorteilhaft gewählt werden aus der Gruppe der verzweigten und unverzweigten Kohlenwasserstoffe und -wachse, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr, sofern die im Hauptanspruch geforderten Bedingungen eingehalten werden.

Erfindungsgemäß vorteilhaft zu verwendende Fett- und/oder Wachskomponenten können aus der Gruppe der pflanzlichen Wachse, tierischen Wachse, Mineralwachse und petrochemischen Wachse gewählt werden. Erfindungsgemäß günstig sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montanwachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse, sofern die im Hauptanspruch geforderten Bedingungen eingehalten werden.

Weitere vorteilhafte Fett- und/oder Wachskomponenten sind chemisch modifizierte Wachse und synthetische

Wachse, wie beispielsweise die unter den Handelsbezeichnungen Syncrowax HRC (Glyceryltribehenat), Syncrowax HGLC ($C_{16-36}$ -Fettsäuretriglycerid) und Syncrowax AW 1C ($C_{18-36}$ -Fettsäure) bei der CRODA GmbH erhältlichen sowie Montanesterwachse, Sasolwachse, hydrierte Jojobawachse, synthetische oder modifizierte Bienenwachse (z. B. Dimethicon Copolyol Bienenwachs und/oder $C_{30-50}$ - Alkyl Bienenwachs), Polyalkylenwachse, Polyethylenglykolwachse, aber auch chemisch modifzierte Fette, wie z. B. hydrierte Pflanzenöle (beispielsweise hydriertes Ricinusöl und/oder hydrierte Cocosfettglyceride), Triglyceride, wie beispielsweise Trihydroxystearin, Fettsäuren, Fettsäureester und Glykolester, wie beispielsweise $C_{20-40}$-Alkylstearat, C20-40-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan sofern die im Hauptanspruch geforderten Bedingungen eingehalten werden.

Erfindungsgemäß können die Fett- und/oder Wachskomponenten sowohl einzeln als auch im Gemisch vorliegen.

Auch beliebige Abmischungen solcher Öl- und Wachskomponenten sind vorteilhaft im Sinne der vorliegenden Erfindung einzusetzen.

Vorteilhaft wird die Ölphase gewählt aus der Gruppe 2-Ethylhexylisostearat, Octyldodecanol, Isotridecylisononanoat, Butylen Glycol Dicaprylat/Dicaprat, 2-Ethylhexylcocoat, C12-15-Alkylbenzoat, Capryl-Caprinsäure-triglycerid, Dicaprylylether sofern die im Hauptanspruch geforderten Bedingungen eingehalten werden.

Besonders vorteilhaft sind Mischungen aus Octyldodecanol, Capryl-Caprinsäure-triglycerid, Dicaprylylether, Dicaprylyl Carbonat, Cocoglyceriden, oder Mischungen aus C12-15-Alkybenzoat und 2-Ethylhexylisostearat, Mischungen aus C12-15-Alkybenzoat und Butylen Glycol Dicaprylat/Dicaprat sowie Mischungen aus C12-15-Alkybenzoat, 2-Ethylhexylisostearat und Isotridecylisononanoat sofern die im Hauptanspruch geforderten Bedingungen eingehalten werden.

Von den Kohlenwasserstoffen sind Paraffinöl, Cycloparaffin, Squalan, Squalen, hydriertes Polyisobuten bzw. Polydecen vorteilhaft im Sinne der vorliegenden Erfindung zu verwenden. sofern die im Hauptanspruch geforderten Bedingungen eingehalten werden.

Erfindungsgemäße W/O-Emulsionen können vorteilhaft mit Hilfe der üblichen W/O - Emulgatoren, gewünschtenfalls unter Zuhilfenahme von O/W-Emulgatoren bzw. weiteren Coemulgatoren hergestellt werden.

W/O-Emulsionen entsprechend der vorliegenden Erfindung enthalten gewünschtenfalls ferner einen oder mehrere Emulgatoren, gewünschtenfalls vorteilhaft gewählt aus der Gruppe der folgenden Substanzen, die in der Regel als W/O-Emulgatoren wirken:

Lecithin, Lanolin, mikrokristallines Wachs (Cera microcristallina) im Gemisch mit Paraffinöl (Paraffinum liquidum), Ozokerit, hydriertem Ricinusöl, Polyglyceryl-3-Oleat, Wollwachssäuregemische, Wollwachsalkoholgemische, Pentaerythrithylisostearat, Polyglyceryl-3-Diisostearat, Bienenwachs (Cera alba) und Stearinsäure, Natriumdihydroxycetylphosphat im Gemisch mit Isopropylhydroxycetylether, Methylglucosedioleat, Methylglucosedioleat im Gemisch mit Hydroxystearat und Bienenwachs, Mineralöl im Gemisch mit Petrolatum und Ozokerit und Glyceryloleat und Lanolinalkohol, Petrolatum im Gemisch mit Ozokerit und hydriertem Ricinusöl und Glycerylisostearat und Polyglyceryl-3-oleat, PEG-7-hydriertes Ricinusöl, Ozokerit und hydriertem Ricinusöl, Polyglyceryl-4-isostearat, Polyglyceryl-4-isostearat im Gemisch mit Cetyldimethiconcopolyol und Hexyllaurat, Laurylmethiconcopolyol, Cetyldimethiconcopolyol, Acrylat/$C_{10-30}$-Alkylacrylat-Crosspolymer, Poloxamer 101, Polyglyceryl-2-dipolyhydroxystearat, Polyglyceryl-3-Diisostearat, Polyglyceryl-4-dipolyhydroxystearat, PEG-30-dipolyhydroxystearat, Diisostearoylpolyglyceryl-3-diisostearat, Polyglyceryl-2-dipolyhydroxystearat, Polyglyceryl-3-dipolyhydroxystearat, Polyglyceryl-4-dipolyhydroxystearat, Polyglyceryl-3-dioleat.

W/O-Emulsionen entsprechend der vorliegenden Erfindung enthalten gewünschtenfalls einen oder mehrere Coemulgatoren, insbesondere vorteilhaft gewählt aus der Gruppe der folgenden Substanzen, die in der Regel als O/W-Emulgatoren wirken:

Glycerylstearat im Gemisch mit Ceteareth-20, Ceteareth-25, Ceteareth-6 im Gemisch mit Stearylalkohol, Cetylstearylalkohol im Gemisch mit PEG-40-Ricinusöl und Natriumcetylstearylsulfat, Triceteareth-4 Phosphat, Natriumcetylstearylsulfat, Lecithin Trilaureth-4 Phosphat, Laureth-4 Phosphat, Stearinsäure, Propylenglycolstearat SE, PEG-25-hydriertes Ricinusöl, PEG-54-hydriertes Ricinusöl, PEG-6 Caprylsäure/Caprinsäureglyceride, Glyceryloleat im Gemisch mit Propylenglycol, Ceteth-2, Ceteth-20, Polysorbat 60, Glycerylstearat im Gemisch mit PEG-100 Stearat, Laureth-4, Ceteareth-3, Isostearylglycerylether, Cetylstearylalkohol im Gemisch mit Natrium Cetylstearylsulfat, Laureth-23, Steareth-2, Glycerylstearat im Gemisch mit PEG-30 Stearat, PEG-40-Stearat, Glycol Distearat, PEG-22-Dodecyl Glycol Copolymer, Polyglyceryl-2-PEG-4-Stearat, Ceteareth-20, Methylglucosesesquistearat, Steareth-10, PEG-20-Stearat, Steareth-2 im Gemisch mit PEG-8 Distearat, Steareth-21, Steareth-20, Isosteareth-20, PEG-45/ Dodecylglycol-Copolymer, Methoxy-PEG-22/Dodecylglycol-Copolymer, PEG-20-Glycerylstearat, PEG-20-Glycerylstearat, PEG-8-Bienenwachs, Polyglyceryl-2-laurat, Isostearyldiglycerylsuccinat, Stearamidopropyl-PGdimoniumchloridphosphat, Glycerylstearat SE, Ceteth-20, Triethylcitrat, PEG-20-Methylglucosesesquistearat, Ceteareth-12, Glycerylstearatcitrat, Cetylphosphat, Triceteareth-4-Phosphat, Trilaureth-4-Phosphat, Polyglycerylmethylglucosedistearat, Kaliumcetylphosphat, Isosteareth-10, Polyglyceryl-2-sesquiisostearat, Ceteth-10, Oleth-20, Isoceteth-20, Glycerylstearat im Gemisch mit Ceteareth-20, Ceteareth-12, Cetylstearylalkohol und Cetylpalmitat, Cetylstearylalkohol im Gemisch mit PEG-20 Stearat, PEG-30-Stearat, PEG-40-Stearat, PEG-100-Stearat.

Es kann auch vorteilhaft im Sinne der vorliegenden Erfindung sein, insbesondere dann, wenn die Ölphase der Zubereitungen wenigstens teilweise aus Siliconölen besteht, Siliconemulgatoren zu verwenden. Die Siliconemulgatoren können vorteilhaft aus der Gruppe grenzflächenaktive Substanzen aus der Gruppe der Alkylmethiconcopolyole und/oder Alkyl-Dimethiconcopolyole gewählt werden, insbesondere aus der Gruppe der Verbindungen, welche gekennzeichnet sind durch die folgende chemische Struktur:

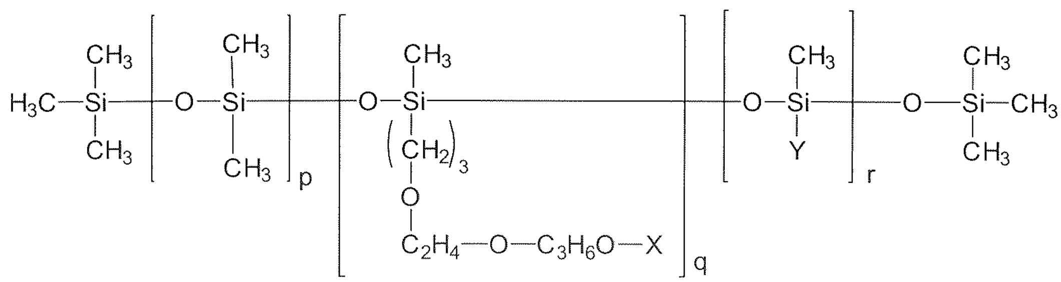

bei welcher X und Y unabhängig voneinander gewählt werden aus der Gruppe H sowie der verzweigten und unverzweigten Alkylgruppen, Acylgruppen und Alkoxygruppen mit 1 - 24 Kohlenstoffatomen, p eine Zahl von 0 - 200 darstellt, q eine Zahl von 1 - 40 darstellt, und r eine Zahl von 1 - 100 darstellt.

Ein Beispiel für besonders vorteilhaft im Sinne der vorliegenden Erfindung zu verwendende Silikonemulgatoren sind Dimethiconcopolyole, welche von der Gesellschaft Th.Goldschmidt AG unter den Warenbezeichnungen ABIL® B 8842, ABIL® B 8843, ABIL® B 8847, ABIL® B 8851, ABIL® B 8852, ABIL® B 8863, ABIL® B 8873 und A-BIL® B 88183 verkauft werden.

Ein weiteres Beispiel für besonders vorteilhaft im Sinne der vorliegenden Erfindung zu verwendende grenzflächenaktiven Substanzen ist das Cetyl Dimethiconcopolyol, welches von der Gesellschaft Th.Goldschmidt AG unter der Warenbezeichnung ABIL® EM 90 verkauft wird.

Ein weiteres Beispiel für besonders vorteilhaft im Sinne der vorliegenden Erfindung zu verwendende grenzflächenaktiven Substanzen ist das Cyclomethicon Dimethiconcopolyol, welches von der Gesellschaft Th. Goldschmidt AG unter der Warenbezeichnung ABIL® EM 97 verkauft wird.

Weiterhin hat sich als ganz besonders vorteilhaft der Emulgator Laurylmethiconcopolyol herausgestellt, welcher unter der Warenbezeichnung Dow Corning® 5200 Formulation Aid von der Gesellschaft Dow Corning Ltd. erhältlich ist.

Die Gesamtmenge an erfindungsgemäß vorteilhaft verwendeten Siliconemulgatoren in den erfindungsgemäßen kosmetischen oder dermatologischen Zubereitungen wird vorteilhaft aus dem Bereich von 0,1 - 10,0 Gew.-%, bevorzugt 0,5 - 5,0 Gew.-% gewählt, bezogen auf das Gesamtgewicht der Zubereitungen.

Erfindungsgemäße Emulsionen im Sinne der vorliegenden Erfindung, z.B. in Form einer Hautschutzcrème, einer Hautlotion, einer kosmetischen Milch, beispielsweise in Form einer Sonnenschutzcreme oder einer Sonnenschutzmilch, sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Ebenso wie Emulsionen von flüssiger und fester Konsistenz als kosmetische Reinigungslotionen oder Reinigungscremes Verwendung finden, können auch die erfindungsgemäßen Zubereitungen versprühbare Reinigungszubereitungen ("Reinigungssprays") darstellen, welche beispielsweise zum Entfernen von Schminken und/oder Make-up oder als milde Waschlotion - ggf. auch für unreine Haut - verwendet werden. Derartige Reinigungszubereitungen können vorteilhaft ferner als sogenannte "rinseoff-Präparate" angewendet werden, welche nach der Anwendung von der Haut abgespült werden

Es ist dem Fachmanne natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Selbstbräunungssubstanzen, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung

zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Es ist ebenfalls von Vorteil, von den erfindungsgemäßen Eigenschaften in Form von dekorativen Kosmetika (Make-Up-Formulierungen) Gebrauch zu machen.

Günstig sind auch solche kosmetischen und dermatologischen Zubereitungen, die in der Form eines Sonnenschutzmittels vorliegen. Vorzugsweise enthalten diese neben dem erfindungsgemäß verwendeten Wirkstoff zusätzlich mindestens eine weitere UVA-Filtersubstanz und/oder mindestens eine UVB-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden beispielsweise in Tagescrèmes gewöhnlich UV-A- bzw. UV-B-Filtersubstanzen eingearbeitet.

Vorteilhafte Breitbandfilter oder UV-B-Filtersubstanzen sind beispielsweise Bis-Resorcinyltriazinderivate mit der folgenden Struktur:

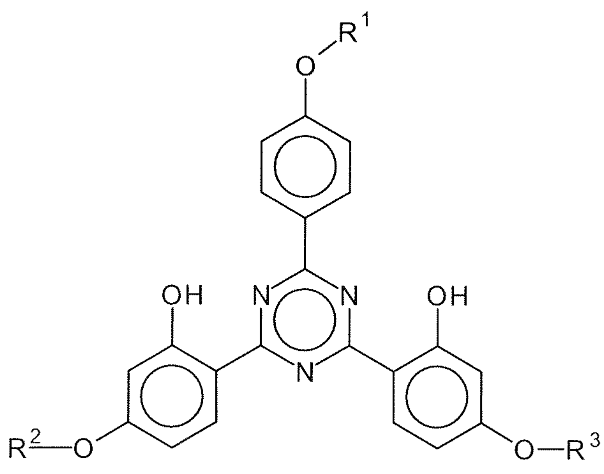

wobei $R^1$, $R^2$ und $R^3$ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethylhexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist.

Auch andere UV-Filtersubstanzen, welche das Strukturmotiv

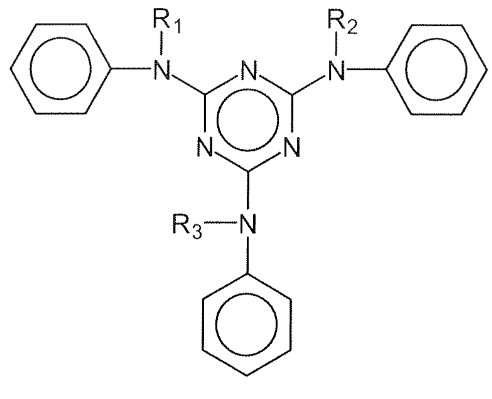

aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel

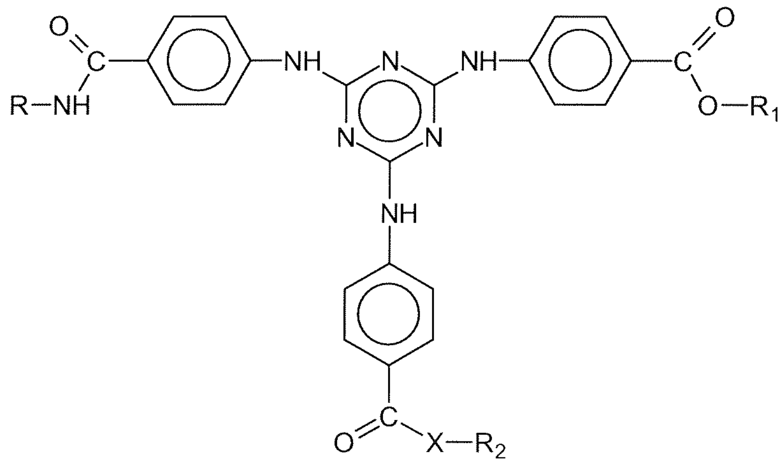

wiedergegeben wird, wobei

R einen verzweigten oder unverzweigten $C_1$-$C_{18}$-Alkylrest, einen $C_5$-$C_{12}$Cycloalkylrest, gegebenenfalls substituiert mit einer oder mehreren $C_1$-$C_4$- Alkylgruppen, darstellt,

X ein Sauerstoffatom oder eine NH-Gruppe darstellt,

$R_1$ einen verzweigten oder unverzweigten $C_1$-$C_{18}$-Alkylrest, einen $C_5$-$C_{12}$Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

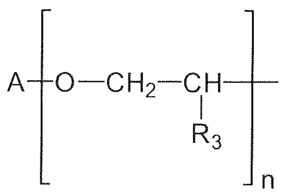

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,

$R_3$ ein Wasserstoffatom oder eine Methylgruppe darstellt,

n eine Zahl von 1 bis 10 darstellt,

$R_2$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt, wenn X die NH-Gruppe darstellt, und

einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

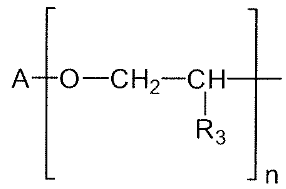

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,

$R_3$ ein Wasserstoffatom oder eine Methylgruppe darstellt,

n eine Zahl von 1 bis 10 darstellt,

wenn X ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel

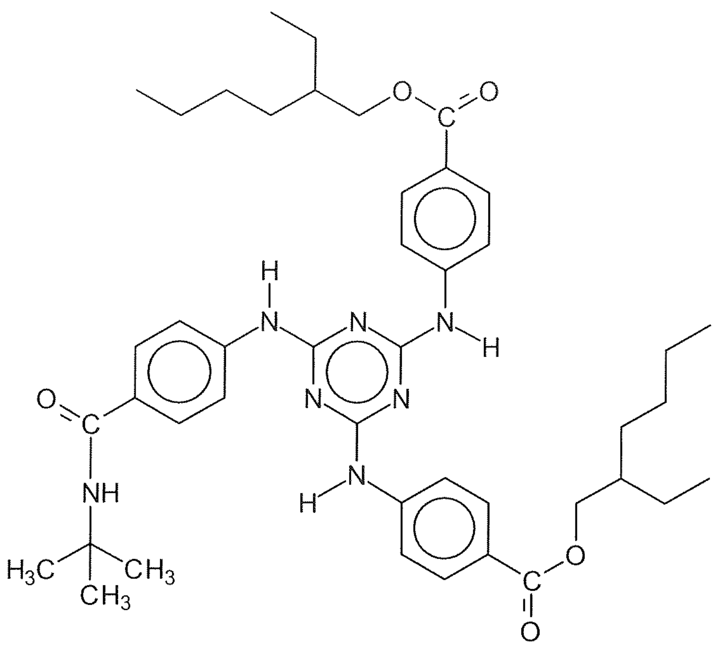

wiedergegeben wird, welches im Folgenden auch als Dioctylbutylamidotriazon (INCI: Dioctylbutamidotriazone) be zeichnet wird und unter der Handelsbezeichnung UVASORB HEB bei Sigma 3V erhältlich ist.

Vorteilhaft im Sinne der vorliegenden Erfindung ist auch ein symmetrisch substituiertes s-Triazin, das 4,4',4''-(1,3,5-Triazin-2,4,6-triyltriimino)-tris-benzoesäure-tris(2-ethylhexyl-ester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Auch in der Europäischen Offenlegungsschrift 775 698 werden bevorzugt einzusetzende Bis-Resorcinyltriazinderivate beschrieben, deren chemische Struktur durch die generische Formel

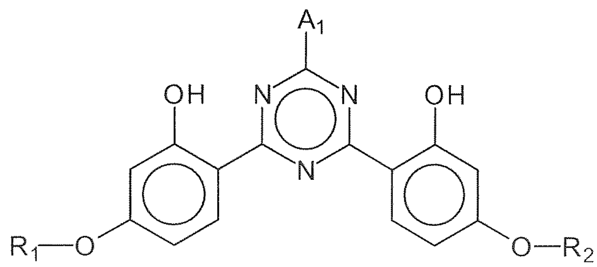

wiedergegeben wird, wobei $R_1$ , $R_2$ und $A_1$ verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxypropyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxypropyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das "2,4-Bis-{[4-(1',1',1',3',5',5',5'-Heptamethylsiloxy-2"-methylpropyloxy)-2-hydroxy]-phenyl}-6-(4-methoxyphenyl)-1,3,5-triazin.

Die Gesamtmenge an einem oder Triazinderivaten in den fertigen kosmetischen oder dermatologischen Zubereitungen wird vorteilhaft aus dem Bereich 0,01 Gew.-% bis 15 Gew.-%, vorzugsweise von 0,1 bis 10 Gew.-% gewählt, jeweils bezogen auf das Gesamtgewicht der Zubereitungen.

Vorteilhafte sulfonierte, wasserlösliche UV-Filter im Sinne der vorliegenden Erfindung sind:

Phenylen-1,4-bis-(2-benzimidazyl)-3,3'-5,5'-tetrasulfonsäure, welche sich durch folgende Struktur auszeichnet:

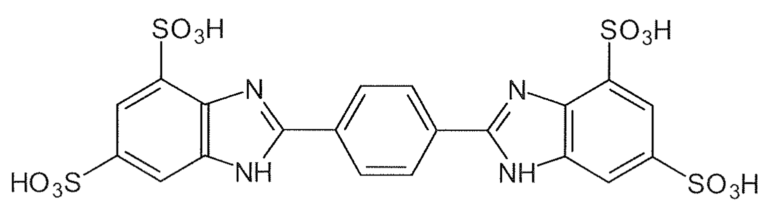

Sowie ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz

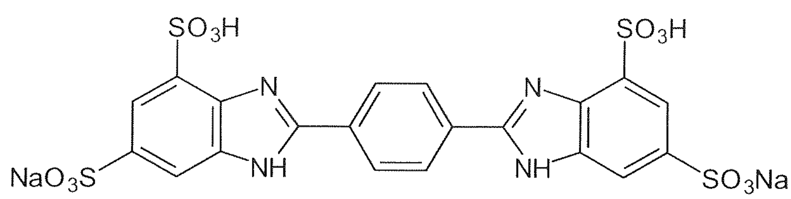

mit der INCI-Bezeichnung Bisimidazylate (CAS-Nr.: 180898-37-7), welches beispielsweise unter der Handelsbezeichnung Neo Heliopan AP bei Haarmann & Reimer erhältlich ist.

Ein weiterer im Sinne der vorliegenden Erfindung vorteilhafter sulfonierter UV-Filter sind die Salze der 2-Phenylbenzimidazol-5-sulfonsäure, wie ihr Natrium-, Kalium- oder ihr Triethanolammonium-Salz, sowie die Sulfonsäure selbst

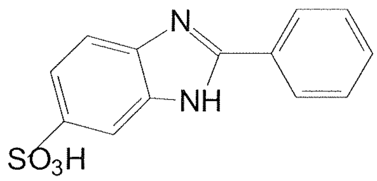

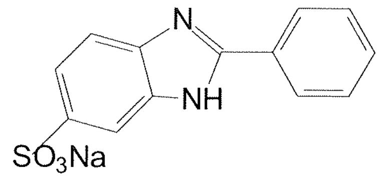

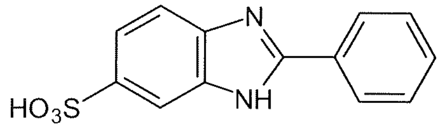

mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist.

Eine weiterer vorteilhafter sulfonierter UV-Filter ist die 3,3'-(1,4-Phenylendimethylene) bis (7,7-dimethyl-2-oxo-bicyclo-[2.2.1] hept-1-ylmethane Sulfonsäure, wie ihr Natrium-, Kalium- oder ihr Triethanolammonium-Salz, sowie die Sulfonsäure selbst:

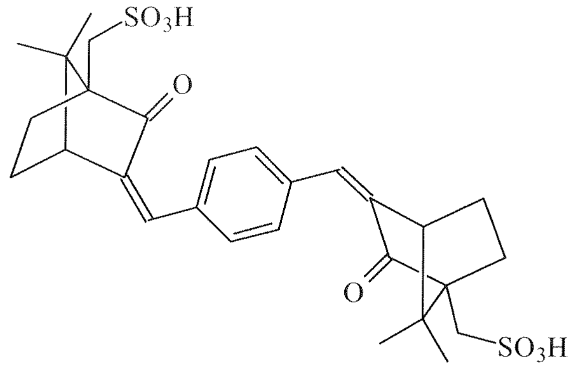

mit der INCI-Bezeichnung Terephtalidene Dicampher Sulfonsäure (CAS.-Nr.: 90457-82-2), welche beispielsweise unter dem Handelsnamen Mexoryl SX von der Fa. Chimex erhältlich ist.

Weitere vorteilhafte wasserlösliche UV-B- und/oder Breitband-Filtersubstanzen sind z. B.:

- Sulfonsäure-Derivate des 3-Benzylidencamphers, wie z. B. 4-(2-Oxo-3-bornylidenme-thyl)benzolsulfonsäure, 2-Methyl-5-(2-oxo-3-bornylidenmethyl)sulfonsäure und deren Salze.

Die Gesamtmenge an einer oder mehreren sulfonierten UV-Filtersubstanzen in den fertigen kosmetischen oder dermatologischen Zubereitungen wird vorteilhaft aus dem Bereich 0,01 Gew.-% bis 20 Gew.-%, vorzugsweise von 0,1 bis 10 Gew.-% gewählt, jeweils bezogen auf das Gesamtgewicht der Zubereitungen.

Ferner vorteilhaft sind das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird und sich durch die folgende Struktur auszeichnet:

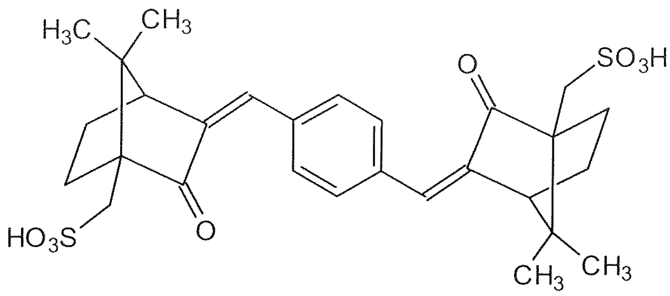

Die UV-B- und/oder Breitband-Filter können öllöslich oder wasserlöslich sein. Vorteilhafte öllösliche UV-B- und/oder Breitband-Filtersubstanzen sind z. B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
- sowie an Polymere gebundene UV-Filter.

Homomenthylsalicylat (INCI: Homosalate) zeichnet sich durch die folgende Struktur aus:

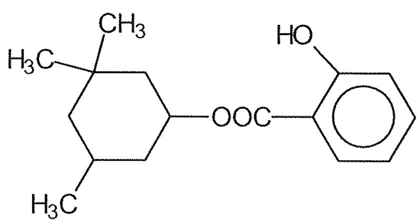

2-Ethylhexyl-2-cyano-3,3-diphenylacrylat (INCI: Octocrylene) ist von BASF unter der Bezeichnung Uvinul® N 539 erhältlich und zeichnet sich durch folgende Struktur aus:

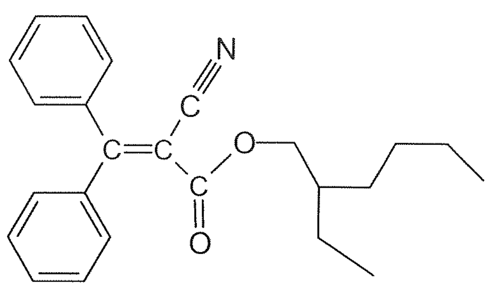

2-Ethylhexyl-2-hydroxybenzoat (2-Ethylhexylsalicylat, Octylsalicylat, INCI: Octyl Salicylate) ist beispielsweise bei Haarmann & Reimer unter der Handelsbezeichnung Neo Heliopan OS erhältlich und zeichnet sich durch die folgende Struktur aus:

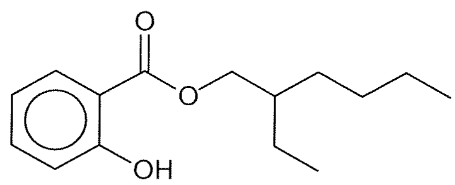

4-Methoxyzimtsäure(2-ethylhexyl)ester (2-Ethylhexyl-4-methoxycinnamat, INCI: Octyl Methoxycinnamate) ist beispielsweise bei Hoffmann-La Roche unter der Handelsbezeichnung Parsol MCX erhältlich und zeichnet sich durch die folgende Struktur aus:

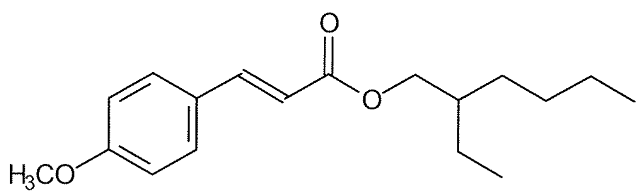

4-Methoxyzimtsäureisopentylester (Isopentyl-4-methoxycinnamat, INCI: Isoamyl p-Methoxycinnamate) ist beispielsweise bei Haarmann & Reimer unter der Handelsbezeichnung Neo Heliopan E 1000 erhältlich und zeichnet sich durch die folgende Struktur aus:

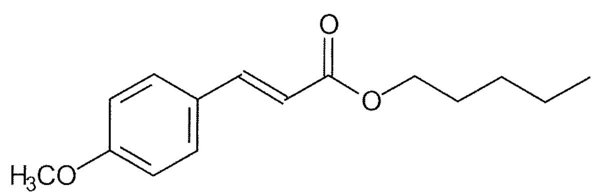

Eine weitere vorteilhafte, bei Raumtemperatur flüssige UV-Filter Substanz im Sinne der vorliegenden Erfindung (3-(4-(2,2-bis-Ethoxycarbonylvinyl)-phenoxy) propenyl)- methyls i-loxan/Dimethylsiloxan Copolymer, welches beispielsweise bei Hoffmann-La Roche unter der Handelsbezeichnung Parsol SLX erhältlich ist.
Die Gesamtmenge an einer oder mehreren bei Raumtemperatur flüssigen UV-Filtersubstanzen in den fertigen kosmetischen oder dermatologischen Zubereitungen wird vorteilhaft aus dem Bereich 0,1 Gew.-% bis 30 Gew.-%, vorzugsweise von 0,5 bis 20 Gew.-% gewählt, jeweils bezogen auf das Gesamtgewicht der Zubereitungen.
Vorteilhafte Dibenzoylmethanderivate im Sinne der vorliegenden Erfindung sind, insbesondere das 4-(tert. -Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird zeichnet sich durch folgende Struktur aus:
Ein weiters vorteilhaftes Dibenzoylmethanderivat ist das 4-Isopropyl-Dibenzoylmethan

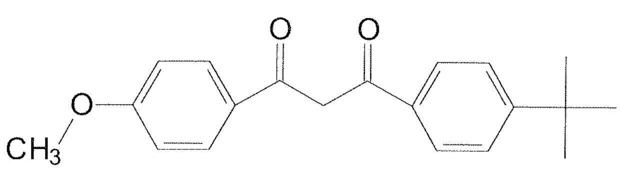

(CAS-Nr. 63250-25-9), welches von Merck unter dem Namen Eusolex 8020 verkauft wird. Das Eusolex 8020 zeichnet sich durch folgende Sturktur aus:

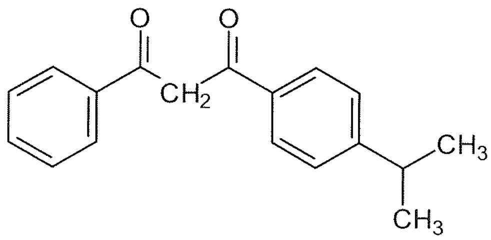

Benzotriazole zeichnen sich durch die folgende Strukturformel aus:

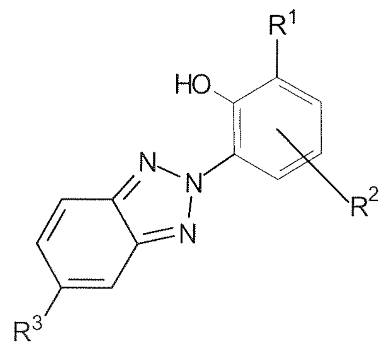

,

worin

- $R^1$ und $R^2$ unabhängig voneinander lineare oder verzweigte, gesättigte oder ungesättigte, substituierte (z. B. mit einem Phenylrest substituierte) oder unsubstituierte Alkylreste mit 1 bis 18 Kohlenstoffatomen und/oder Polymerreste, welche selbst nicht UV-Strahlen absorbieren (wie z. B. Silikonreste, Acrylatreste und dergleichen mehr), darstellen können und
- $R_3$ aus der Gruppe H oder Alkylrest mit 1 bis 18 Kohlenstoffatomen gewählt wird.

Ein vorteilhaftes Benzotriazol im Sinne der vorliegenden Erfindung ist das 2,2'-Methylenbis-(6-(2H-benzotriazol-2-yl)-4-(1,1,3,3-tetramethylbutyl)-phenol), ein Breitbandfilter, welcher durch die chemische Strukturformel

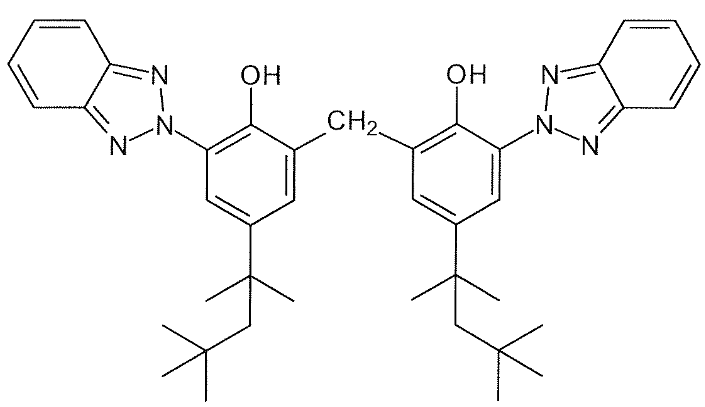

gekennzeichnet ist und unter der Handelsbezeichnung Tinosorb® M bei der CIBA-Chemikalien GmbH erhältlich ist.
Vorteilhaftes Benzotriazol im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]-propyl]-phenol (CAS-Nr.: 155633-54-8) mit der INCI-Bezeichnung Drometrizole Trisiloxane, welches durch die chemische Strukturformel

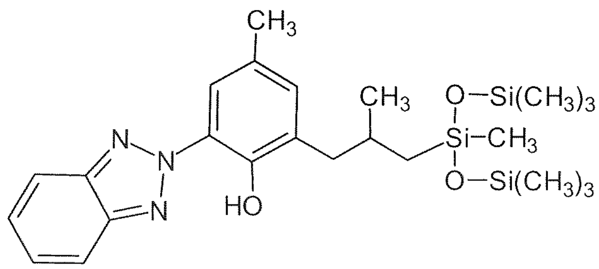

gekennzeichnet ist.

Weitere vorteilhafte Benzotriazole im Sinne der vorliegenden Erfindung sind [2,4'-Dihydroxy-3-(2H-benzotriazol-2-yl)-5-(1,1,3,3-tetramethylbutyl)-2'-n-octoxy-5'-benzoyl]diphenylmethan, 2,2' Methylen-bis-[6-(2H-benzotriazol-2-yl)-4-(methyl)phenol], 2,2'-Methylen-bis-[6-(2H-benzotiazol-2-yl)-4-(1,1,3,3-tetramethylbutyl)phenol], 2-(2'-Hydroxy-5'-octylphenyl)-benzotriazol, 2-(2'-Hydroxy-3',5'-di-t-amylphenyl)benzotriazol und 2-(2'-Hydroxy-5'-methylphenyl)benzotriazol.

Erfindungsgemäß vorteilhaft enthalten kosmetische oder dermatologische Zubereitungen 0,1 bis 20 Gew.-%, vorteilhaft 0,5 bis 15 Gew.-%, ganz besonders bevorzugt 0,5 bis 10 Gew.-% eines oder mehrerer Benzotriazole.

Erfindungsgemäße kosmetische und dermatologische Zubereitungen enthalten ferner vorteilhaft, wenngleich nicht zwingend, anorganische Pigmente auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$), Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Diese Pigmente sind röntgenamorph oder nicht-röntgenamorph. Besonders bevorzugt handelt es sich um Pigmente auf der Basis von $TiO_2$.

Röntgenamorphe Oxidpigmente sind Metalloxide oder Halbmetalloxide, welche bei Röntgenbeugungsexperimenten keine oder keine erkenntliche Kristallstruktur erkennen lassen. Oftmals sind solche Pigmente durch Flammenreaktion erhältlich, beispielsweise dadurch, daß ein Metall- oder Halbmetallhalogenid mit Wasserstoff und Luft (oder reinem Sauerstoff) in einer Flamme umgesetzt wird.

In kosmetischen, dermatologischen oder pharmazeutischen Formulierungen werden röntgenamorphe Oxidpigmente als Verdickungs- und Thixotropierungsmittel, Fließhilfsmittel, zur Emulsions- und Dispersionsstabilisierung und als Trägersubstanz (beispielsweise zur Volumenerhöhung von feinteiligen Pulvern oder Pudern) eingesetzt.

Bekannte und in der kosmetischen oder dermatologischen Galenik oftmals verwendete röntgenamorphe Oxidpigmente sind die Siliciumoxide des Typs Aerosil® (CAS-Nr. 7631-86-9. Aerosile®, erhältlich von der Gesellschaft DEGUSSA, zeichnen sich durch geringe Partikelgröße (z.B. zwischen 5 und 40 nm) aus, wobei die Partikel als kugelförmige Teilchen sehr einheitlicher Abmessung anzusehen sind. Makroskopisch sind Aerosile® als lockere, weiße Pulver erkenntlich. Im Sinne der vorliegenden Erfindung sind röntgenamorphe Siliciumdioxidpigmente besonders vorteilhaft, und unter diesen gerade solche des Aerosil®-Typs bevorzugt.

Vorteilhafte Aerosil®-Typen sind beispielsweise Aerosil® OX50, Aerosil® 130, Aerosil® 150, Aerosil® 200, Aerosil® 300, Aerosil® 380, Aerosil® MOX 80, Aerosil® MOX 170, Aerosil® COK 84, Aerosil® R 202, Aerosil® R 805, Aerosil® R 812, Aerosil® R 972, Aerosil® R 974, Aerosil® R976.

Erfindungsgemäß enthalten kosmetische oder dermatologische Lichtschutzzubereitungen 0,1 bis 20 Gew.-%, vorteilhaft 0,5 bis 10 Gew.-%, ganz besonders bevorzugt 1 bis 5 Gew.-% röntgenamorphe Oxidpigmente.

Die nicht-röntgenamorphen anorganischen Pigmente liegen erfindungsgemäß vorteilhaft in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

$$n\ TiO_2 + m\ (RO)_3Si\text{-}R' \rightarrow n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer

durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| Handelsname | Coating | Hersteller |
|---|---|---|
| Z- Cote HP1 | 2% Dimethicone | BASF |
| Z- Cote | / | BASF |
| ZnO NDM | 5% Dimethicone | H&R |
| MZ-505 S | 5% Methicone | Tayca Corp. |

Geeignete Titandioxidpartikel und Vordispersionen von Titandioxidpartikeln sind unter folgenden Handelsbezeichnungen bei den aufgeführten Firmen erhältlich:

| Handelsname | Coating | Hersteller |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul TiO2) | Octyltrimethylsilan | Degussa |

Vorteilhafte TiO2-Pigmente sind beispielsweise unter der Handelsbezeichnung T 805, vorteilhafte TiO2/Fe2O3-Mischoxide unter der Handelsbezeichnung T 817 von der Firma Degussa erhältlich.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 - 30 Gew.-%, bevorzugt 0,1 - 10,0, insbesondere 0,5 - 6,0 Gew.-% gewählt, bezogen auf das Gesamtgewicht der Zubereitungen.

Die erfindungsgemäßen kosmetischen und dermatologischen Zubereitungen können kosmetische Wirk-, Hilfs- und/od er Zusatzstoffe enthalten, wie sie üblicherweise in solchen Zubereitungen verwendet werden, z.B. Antioxidationsmittel, Konservierungsmittel, Bakterizide, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

In der Lebensmitteltechnologie zugelassene Konservierungsmittel, die mit ihrer E-Nummer nachfolgend aufgeführt sind, sind erfindungsgemäß vorteilhaft zu verwenden.

| | | | |
|---|---|---|---|
| E 200 | Sorbinsäure | E 227 | Calciumhydrogensulfit |
| E 201 | Natriumsorbat | E 228 | Kaliumhydrogensulfit) |
| E 202 | Kaliumsorbat | E 230 | Biphenyl (Diphenyl) |
| E 203 | Calciumsorbat | E 231 | Orthophenylphenol |
| E 210 | Benzoesäure | E 232 | Natriumorthophenylphenolat |
| E 211 | Natriumbenzoat | E 233 | Thiabendazol |
| E 212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Calciumformiat |
| E 216 | p-Hydroxybenzoesäure-n-propylester | E 239 | Hexamethylentetramin |

(fortgesetzt)

| | | | |
|---|---|---|---|
| E 217 | p-Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |
| E 221 | Natriumsulfit | E 280 | Propionsäure |
| E 222 | Natriumyhdrogensulfit | E 281 | Natriumpropionat |
| E 223 | Natriumdisulfit | E 282 | Calciumpropionat |
| E 224 | Kaliumdisulfit | E 283 | Kaliumpropionat |
| E 226 | Calciumsulfit | E 290 | Kohlendioxid |

Ferner sind erfindungsgemäß in der Kosmetik gebräuchliche Konervierungsmittel oder Konservierungshilfsstoffe Dibromdicyanobutan (2-Brom-2-brommethylglutarodinitril), 3-Iod-2-propinylbutylcarbamat, 2-Brom-2-nitro-propan-1,3-diol, Imidazolidinylharnstoff, 5-Chlor-2-methyl-4-isothiazolin-3-on, 2 -Chloracetamid, Benzalkoniumchlorid, Benzylalkohol geeignet. Formaldehydabspalter.

Ferner sind Phenylhydroxyalkylether, insbesondere die unter der Bezeichnung Phenoxyethanol bekannte Verbindung aufgrund ihrer bakteriziden und fungiziden Wirkungen auf eine Anzahl von Mikroorganismen als Konservierungsmittel geeignet.

Auch andere keimhemmende Mittel sind ebenfalls geeignet, in die erfindungsgemäßen Zubereitungen eingearbeitet zu werden. Vorteilhafte Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatrien-1-ol) sowie die in den Patentoffenlegungsschriften DE-37 40 186, DE-39 38 140, DE-42 04 321, DE-42 29 707, DE-43 09 372, DE-44 11 664, DE-195 41 967, DE-195 43 695, DE-195 43 696, DE-195 47 160, DE-196 02 108, DE-196 02 110, DE-196 02 111, DE-196 31 003, DE-196 31 004 und DE-196 34 019 und den Patentschriften DE-42 29 737, DE-42 37 081, DE-43 24 219, DE-44 29 467, DE-44 23 410 und DE-195 16 705 beschriebenen Wirkstoffe bzw. Wirkstoffkombinationen. Auch Natriumhydrogencarbonat ist vorteilhaft zu verwenden.

Es ist darüberhinaus vorteilhaft, den Zubereitungen im Sinne der vorliegenden Erfindung weitere antiirritative oder antientzündliche Wirkstoffe zuzugeben, insbesondere Batylalkohol (α-Octadecylglycerylether), Selachylalkohol (α-9-Octadecenylglyceryl-ether), Chimylalkohol (α-Hexadecylglycerylether), Bisabolol und/oder Panthenol.

Es ist ebenfalls vorteilhaft, den Zubereitungen im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Carnosin, D-Carnosin, L-Carnosin und deren Derivate (z.B. Anserin), Carotinoide, Carotine (z.B. α-Carotin, β-Carotin, ψ-Lycopin) und deren Derivate, Chlorogensäure und deren Derivate, Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallen extrakte, Bilirubin, Biliverdin, EDTA, EGTA und deren Derivate, Iminodisuccinat, ungesättigte Fettsäuren und deren Derivate (z.B. γ-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoeharzes, Propylgallat, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 0,1 - 5 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Zubereitungen gemäß der vorliegenden Erfindung können auch Verwendung als Grundlage für kosmetische oder dermatologische Desodorantien bzw. Antitranspirantien finden. Alle für Desodorantien bzw. Antitranspirantien gängigen Wirkstoffe können vorteilhaft genutzt werden, beispielsweise Geruchsüberdecker wie die gängigen Parfümbestandteile, Geruchsabsorber, beispielsweise die in der Patentoffenlegungsschrift DE-P 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure.

Die Menge solcher Wirkstoffe (eine oder mehrere Verbindungen) in den Zubereitungen gemäß der Erfindung beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die Wasserphase der kosmetischen Zubereitungen im Sinne der vorliegenden Erfindung kann auch Gelcharakter aufweisen, die neben einem wirksamen Gehalt am erfindungsgemäß eingesetzten Substanzen und dafür üblicherweise verwendeten Lösungsmitteln, bevorzugt Wasser, noch weitere organische und/oder anorganische Verdikkungsmittel.

Das oder die anorganischen Verdickungsmittel können beispielsweise vorteilhaft gewählt werden aus der Gruppe der modifizierten oder unmodifizierten, natürlich vorkommender oder synthetischer Schichtsilikate.

Es ist zwar durchaus günstig, reine Komponenten einzusetzen, es können jedoch auch in vorteilhafter Weise, Gemische verschiedener modifizierter und/oder unmodifizierter Schichtsilicate den erfindungsgemäßen Zusammensetzungen einzuverleiben.

Unter Schichtsilicaten, welche auch Phyllosilicate genannt werden, sind im Rahmen dieser Anmeldung Silicate und Alumosilicate zu verstehen, in welchen die Silicat- bzw. Aluminateinheiten über drei Si-O- oder Al-O- Bindungen untereinander verknüpft sind und eine gewellte Blatt- oder Schichtenstruktur ausbilden. Die vierte Si-O- bzw. Al-O-Valenz wird durch Kationen abgesättigt. Zwischen den einzelnen Schichten bestehen schwächere elektrostatische Wechselwirkungen, z.B. Wasserstoffbrückenbindungen. Das Schichtgefüge indessen ist weitgehend durch starke, kovalente Bindungen geprägt.

Die Stöchiometrie der Blattsilicate ist
$(Si_2O_5{}^{2-})$ für reine Silicatstrukturen und
$(Al_mSi^{2-}{}_mO_5{}^{(2+m)-})$ für Alumosilicate.
m ist eine Zahl größer als Null und kleiner als 2.

Liegen keine reinen Silicate sondern Alumosilicate vor, ist dem Umstande Rechnung zu tragen, daß jede durch $Al^{3+}$ ersetzte $Si^{4+}$ - Gruppe ein weiteres einfach geladenes Kation zur Ladungsneutralisierung erfordert.

Die Ladungsbilanz wird bevorzugt durch $H^+$, Alkali- oder Erdalkalimetallionen ausgeglichen. Auch Aluminium als Gegenion ist bekannt und vorteilhaft. Im Gegensatz zu den Alumosilicaten werden diese Verbindungen Aluminiumsilicate genannt. Auch "A-luminiumalumosilicate", in welchen Aluminium sowohl im Silicatnetz, als auch als Gegenion vorliegt, sind bekannt und für die vorliegende Erfindung gegebenenfalls von Vorteil.

Schichtsilicate sind in der Literatur gut dokumentiert, z.B. im "Lehrbuch der Anorganischen Chemie", A.F. Hollemann, E. Wiberg und N. Wiberg, 91.-100. Aufl., Walter de Gruyter - Verlag 1985, passim, sowie "Lehrbuch der Anorganischen Chemie", H.Remy, 12. Aufl., Akademische Verlagsgesellschaft, Leipzig 1965, passim. Die Schichtenstruktur von Montmorillonit ist Römpps Chemie-Lexikon, Franckh'sche Verlagshandlung W. Keller & Co., Stuttgart, 8.Aufl., 1985, S. 2668 f., zu entnehmen.

Beispiele für Schichtsilicate sind:

| | |
|---|---|
| Montmorillonit | $Na_{0,33}((Al_{1,67}Mg_{0,33})(OH)_2(S_{i4}O_{10}))$ |
| oft vereinfacht: | $Al_2O_3{*}4SiO_2{*}H_2O{*}nH_2O$ bzw. $Al_2[(OH)_2/Si_4O_{10}] \cdot n\ H_2O$ |
| Kaolinit | $Al_2(OH)_4(Si_2O_5)$ |
| Ilit | $(K, H_3O)_y(Mg_3(OH)_2(Si_{4-y}Al_yO_{10}))$ |
| und | $(K, H_3O)_y(Al_2(OH)_2(Si_{4-y}Al_yO_{10}))$ mit y = 0,7 - 0,9 |
| Beidellit | $(Ca, Na)_{0,3}(Al_2(OH)_2(Al_{0,5}Si_{3,5}O_{10}))$ |
| Nontronit | $Na_{0,33}(Fe_2(OH)_2(Al_{0,33}Si_{3,67}O_{10}))$ |
| Saponit | $(Ca,Na)_{0,33}((Mg,Fe)_3(OH)_2(Al_{0,33}Si_{3,67}O_{10}))$ |

Hectorit $Na_{0,33}((Mg,Li)_3(OH,F)_2(Si_4O_{10}))$

Montmorillonit stellt das Hauptmineral der natürlich vorkommenden Bentonite dar.

Sehr vorteilhafte anorganische Gelbildner im Sinne der vorliegenden Erfindung sind Aluminiumsilikate wie die Montmorillonite (Bentonite, Hectorite sowie deren Derivate wie Quaternium-18 Bentonit, Quaternium-18 Hectorite, Stearalkonium Bentonite bzw. Stearalkonium Hectorite) oder aber Magnesium-Aluminium-Silikate (Veegum®-Typen) sowie Natrium-Magnesium-Silikate (Laponite®-Typen)

Montmorillonite stellen zu den dioktaedrischen Smektiten gehörende Tonmineralien dar und sind in Wasser quellende, aber nicht plastisch werdende Massen. Die Schichtpakete in der Dreischicht-Struktur der Montmorillonite können durch reversible Einlagerung von Wasser (in der 2-7fachen Menge) u. a. Substanzen wie z. B. Alkoholen, Glykolen, Pyridin, $\alpha$-Picolin, Ammonium-Verbindungen, Hydroxy-Aluminosilicat-Ionen usw. aufquellen.

Die oben angegebene chemische Formel ist nur angenähert; da M. ein großes Ionenaustausch-Vermögen besitzt, kann Al gegen Mg, $Fe^{2+}$, $Fe^{3+}$, Zn, Pb (z. B. aus Schadstoffen in Abwässern) Cr, auch Cu und andere ausgetauscht werden. Die daraus resultierende negative Ladung der Oktaeder-Schichten wird durch Kationen, insbesondere $Na^+$ (Natrium-Montmorillonit) und $Ca^{2+}$ (der Calcium-Montmorillonit ist nur sehr wenig quellfähig) in Zwischenschicht-Positionen ausgeglichen.

Im Sinne der vorliegenden Erfindung vorteilhafte synthetische Magnesiumsilikate bzw. Bentonite werden beispielsweise von Süd-Chemie unter der Handelsbezeichung Optigel® vertrieben.

Ein im Sinne der vorliegenden Erfindung vorteilhaftes Aluminiumsilikat wird beispielsweise von der R. T. Vanderbilt Comp., Inc., unter der Handelsbezeichnung Veegum® vertrieben. Die verschiedenen Veegum®-Typen, welche alle erfindungsgemäß vorteilhaft sind, zeichnen sich durch folgende Zusammensetzungen aus

| | (regular grade) | HV | K | HS | S-728 |
|---|---|---|---|---|---|
| $SiO_2$ | 55,5 | 56,9 | 64,7 | 69,0 | 65,3 |
| MgO | 13,0 | 13,0 | 5,4 | 2,9 | 3,3 |
| $Al_2O_3$ | 8,9 | 10,3 | 14,8 | 14,7 | 17,0 |
| $Fe_2O_3$ | 1,0 | 0,8 | 1,5 | 1,8 | 0,7 |
| CaO | 2,0 | 2,0 | 1,1 | 1,3 | 1,3 |
| $Na_2O$ | 2,1 | 2,8 | 2,2 | 2,2 | 3,8 |
| $K_2O$ | 1,3 | 1,3 | 1,9 | 0,4 | 0,2 |
| Veraschungsverlust | 11,1 | 12,6 | 7,6 | 5,5 | 7,5 |

Diese Produkte quellen in Wasser unter Bildung viskoser Gele, welche alkalisch reagieren. Durch Organophilierung von Montmorillonit bzw. Bentoniten (Austausch der Zwischenschicht-Kationen gegen quaternäre Alkylammonium-Ionen) entstehen Produkte (Bentone), die bevorzugt zur Dispergierung in organischen Lösemitteln und Ölen, Fetten, Salben, Farben, Lacken und in Waschmitteln eingesetzt werden.

Bentone® ist eine Handelsbezeichnung für verschiedene neutrale und chemisch inerte Geliermittel, die aus langkettigen, organischen Ammoniumsalzen und speziellen Montmorillonit-Sorten aufgebaut sind. Bentone quellen in organischen Medien und bringen diese zum Quellen. Die Gele sind in verdünnten Säuren und Alkalien beständig, bei längerer Berührung mit starken Säuren und Alkalien verlieren sie ihre Geliereigenschaften jedoch teilweise. Aufgrund ihres organophilen Charakters sind die Bentone nur schwer durch Wasser benetzbar.

Folgende Bentone® -Typen werden beispielsweise von der Gesellschaft Kronos Titan vertrieben: Bentone® 27, ein organisch modifiziertes Montmorillonit, Bentone® 34 (Dimethyldioctylammoniumbentonit), das nach US 2,531,427 hergestellt wird und wegen seiner lipophilen Gruppen besser im lipophilen Medium als in Wasser quillt, Bentone® 38, ein organisch modifiziertes Montmorillonit, ein cremefarbenes bis weißes Pulver, Bentone® LT, ein gereinigtes Tonmineral, Bentone® Gel MIO, ein organisch modifiziertes Montmorillonit, das in Mineralöl (SUS-71) feinst suspendiert angeboten wird (10 % Bentonit, 86,7 % Mineralöl und 3,3 % Netzmittel), Bentone® Gel IPM, ein organisch modifiziertes Bentonit, das in Isopropylmyristat suspendiert ist (10 % Bentonit, 86,7 % Isopropylmyristat, 3,3 % Netzmittel), Bentone® Gel CAO, ein organisch modifiziertes Montmorillonit, das in Ricinusöl aufgenommen ist (10 % Bentonit, 86,7 % Ricinusöl und 3,3 % Netzmittel), Bentone® Gel Lantrol, ein organisch modifiziertes Montmorillonit, das in Pastenform zur Weiterverarbeitung, insbesondere zur Herstellung kosmetischer Mittel bestimmt ist; 10 % Bentonit, 64,9 Lantrol (Wollwachsöl), 22,0 Isopropylmyristat, 3,0 Netzmittel und 0,1 p-Hydroxybenzoesäurepropylester, Bentone® Gel Lan I, eine 10 %ige Bentone® 27-Paste in einer Mischung aus Wollwachs USP und Isopropylpalmitat, Bentone® Gel Lan II, eine Bentonit-Paste in reinem, flüssigem Wollwachs, Bentone® Gel NV, eine 15 %ige Bentone® 27-Paste in Dibutylphthalat, Bentone® Gel OMS, eine Bentonit-Paste in Shellsol T. Bentone® Gel OMS 25, eine Bentonit Paste in Isoparaffinischen Kohlenwasserstoffen (Idopar® H), Bentone® Gel IPP, eine Bentonit-Paste in Isopropylpalmitat.

"Hydrokolloid" ist die technologische Kurzbezeichnung für die an sich richtigere Bezeichnung "hydrophiles Kolloid". Hydrokolloide sind Makromoleküle, die eine weitgehend lineare Gestalt haben und über intermolekulare Wechselwirkungskräfte verfügen, die Neben- und Hauptvalenzbindungen zwischen den einzelnen Molekülen und damit die Ausbildung eines netzartigen Gebildes ermöglichen. Sie sind teilweise wasserlösliche natürliche oder synthetische Polymere, die in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie gleichzeitig die Beweglichkeit des Wassers einschränken. Solche wasserlöslichen Polymere stellen eine große Gruppe chemisch sehr unterschiedlicher natürlicher und synthetischer Polymere dar, deren gemeinsames Merkmal ihre Löslichkeit in Wasser bzw. wäßrigen Medien ist. Voraussetzung dafür ist, daß diese Polymere über eine für die Wasserlöslichkeit ausreichende Anzahl an hydrophilen Gruppen besitzen und nicht zu stark vernetzt sind. Die hydrophilen Gruppen können nichtionischer, anionischer oder kationischer Natur sein, beispielsweise wie folgt:

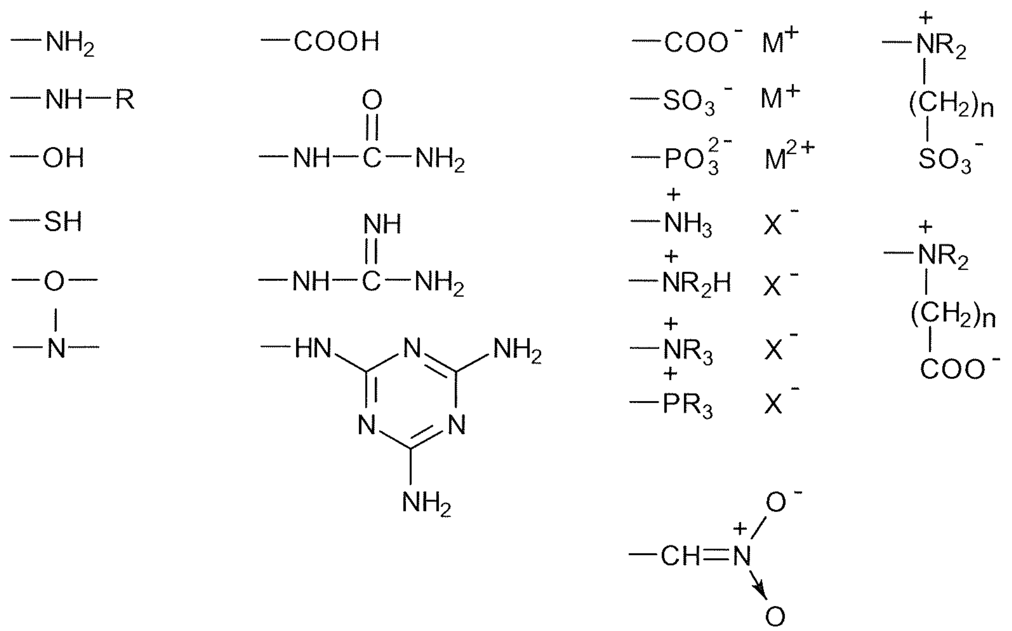

Die Gruppe der kosmetisch und dermatologisch relevanten Hydrokolloide läßt sich wie folgt einteilen in:

- organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein,
- organische, abgewandelte Naturstoffe, wie z. B. Carboxymethylcellulose und andere Celluloseether, Hydroxyethyl- und -propylcellulose und mikrokristalline Cellulose dergleichen,
- organische, vollsynthetische Verbindungen, wie z. B. Polyacryl- und Polymethacryl-Verbindungen, Vinylpolymere, Polycarbonsäuren, Polyether, Polyimine, Polyamide, Polyurethane
- anorganische Verbindungen, wie z. B. Polykieselsäuren, Tonmineralien wie Montmorillonite, Zeolithe, Kieselsäuren.

Mikrokristalline Cellulose ist ein vorteilhaftes Hydrokolloid im Sinne der vorliegenden Erfindung. Sie ist beispielsweise von der "FMC Corporation Food and Pharmaceutical Products" unter der Handelsbezeichnung Avicel® erhältlich. Ein besonders vorteilhaftes Produkt im Sinne der vorliegenden Erfindung ist der Typ Avicel® RC-591, bei dem es sich um modifizierte mikrokristalline Cellulose handelt, die sich zu 89% aus mikrokristalliner Cellulose und zu 11% aus Natrium Carboxymethyl Cellulose zusammensetzt. Weitere Handelsprodukte dieser Rohstoffklasse sind Avicel® RC/ CL, Avicel® CE-15, Avicel® 500.

Weitere erfindungsgemäß vorteilhafte Hydrokolloide sind beispielsweise Methylcellulosen, als welche die Methylether der Cellulose bezeichnet werden. Sie zeichnen sich durch die folgende Strukturformel aus

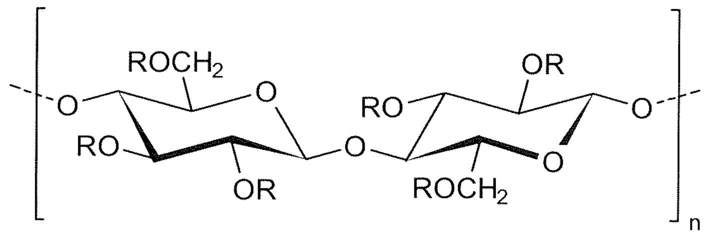

in der R ein Wasserstoff oder eine Methylgruppe darstellen kann.

Insbesondere vorteilhaft im Sinne der vorliegenden Erfindung sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, beispielsweise die unter der Handelsbezeichnung Methocel® E4M bei der Dow Chemical Comp. erhältlichen.

Erfindungsgemäß ferner vorteilhaft ist Natriumcarboxymethylcellulose, das NatriumSalz des Glykolsäureethers der Cellulose, für welches R in Strukturformel I ein Wasserstoff und/oder $CH_2$-COONa darstellen kann. Besonders bevorzugt sind die unter der Handelsbezeichnung Natrosol Plus 330 CS bei Aqualon erhältliche, auch als Cellulose Gum bezeichnete Natriumcarboxymethylcellulose.

Bevorzugt im Sinne der vorliegenden Erfindung ist ferner Xanthan (CAS-Nr. 11138-66-2), auch Xanthan Gummi genannt, welches ein anionisches Heteropolysaccharid ist, das in der Regel durch Fermentation aus Maiszucker gebildet und als Kaliumsalz isoliert wird. Es wird von Xanthomonas campestris und einigen anderen Species unter aeroben Bedingungen mit einem Molekulargewicht von $2\times10^6$ bis $24\times10^6$ produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Xanthan ist die Bezeichnung für das erste mikrobielle anionische Heteropolysaccharid. Es wird von Xanthomonas campestris und einigen anderen Species unter aeroben Bedingungen mit einem Molekulargewicht von 2-15 $10^6$ produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Die Anzahl der Pyruvat-Einheiten bestimmt die Viskosität des Xanthans. Xanthan wird in zweitägigen Batch-Kulturen mit einer Ausbeute von 70-90 %, bezogen auf eingesetztes Kohlenhydrat, produziert. Dabei werden Ausbeuten von 25-30 g/l erreicht. Die Aufarbeitung erfolgt nach Abtöten der Kultur durch Fällung mit z. B. 2-Propanol. Xanthan wird anschließend getrocknet und gemahlen.

Vorteilhafter Gelbildner im Sinne der vorliegenden Erfindung ist ferner Carrageen, ein gelbildender und ähnlich wie Agar aufgebauter Extrakt aus nordatlantischen, zu den Florideen zählenden Rotalgen (Chondrus crispus und Gigartina stellata).

Häufig wird die Bezeichnung Carrageen für das getrocknete Algenprodukt und Carrageenan für den Extrakt aus diesem verwendet. Das aus dem Heißwasserextrakt der Algen ausgefällte Carrageen ist ein farbloses bis sandfarbenes Pulver mit einem Molekulargewichtsbereich von 100 000-800 000 und einem Sulfat-Gehalt von ca. 25 %. Carrageen, das in warmem Wasser sehr leicht lösl. ist; beim Abkühlen bildet sich ein thixotropes Gel, selbst wenn der Wassergehalt 95-98 % beträgt. Die Festigkeit des Gels wird durch die Doppelhelix-Struktur des Carrageens bewirkt . Beim Carrageenan unterscheidet man drei Hauptbestandteile: Die gelbildende κ-Fraktion besteht aus D-Galactose-4-sulfat und 3,6-Anhydro-α-D-galactose, die abwechselnd in 1,3- und 1,4-Stellung glykosidisch verbunden sind (Agar enthält demgegenüber 3,6-Anhydro-α-L-galactose). Die nicht gelierende λ-Fraktion ist aus 1,3-glykosidisch verknüpften D-Galactose-2-sulfat und 1,4-verbundenen D-Galactose-2,6-disulfat-Resten zusammengesetzt u. in kaltem Wasser leicht löslich. Das aus D-Galactose-4-sulfat in 1,3-Bindung und 3,6-Anhydro-α-D-galactose-2-sulfat in 1,4-Bindung aufgebaute$_ι$.-Carrageenan ist sowohl wasserlöslich als auch gelbildend. Weitere Carrageen-Typen werden ebenfalls mit griechischen Buchstaben bezeichnet: α, β, γ, μ, ν, ξ, π, ω, χ. Auch die Art vorhandener Kationen ($K^+$, $NH_4^+$, $Na^+$, $Mg^{2+}$, $Ca^{2+}$) beeinflußt die Löslichkeit der Carrageene.

Polyacrylate sind ebenfalls vorteilhaft im sinne der vorliegenden Erfindung zu verwendende Gelatoren. Erfindungsgemäß vorteilhafte Polyacrylate sind Acrylat-Alkylacrylat-Copolymere, insbesondere solche, die aus der Gruppe der sogenannten Carbomere oder Carbopole (Carbopol® ist eigentlich eine eingetragene Marke der B. F. Goodrich Company) gewählt werden. Insbesondere zeichnen sich das oder die erfindungsgemäß vorteilhaften Acrylat-Alkylacrylat-Copolymere durch die folgende Struktur aus:

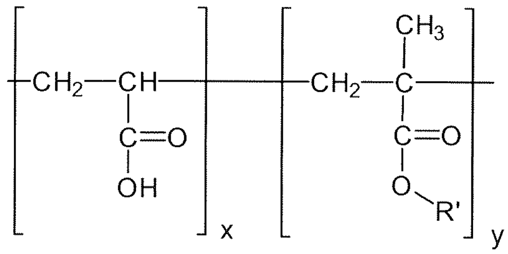

Darin stellen R' einen langkettigen Alkylrest und x und y Zahlen dar, welche den jeweiligen stöchiometrischen Anteil der jeweiligen Comonomere symbolisieren.

Erfindungsgemäß besonders bevorzugt sind Acrylat-Copolymere und/oder Acrylat-Alkylacrylat-Copolymere, welche unter den Handelbezeichnungen Carbopol® 1382, Carbopol® 981 und Carbopol® 5984 von der B. F.Goodrich Company erhältlich sind, bevorzugt Polyacrylate aus der Gruppe der Carbopole der Typen 980, 981, 1382, 2984, 5984 sowie besonders bevorzugt Cabopol Ultrez.

Ferner vorteilhaft sind Copolymere aus $C_{10-30}$-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Vorteilhaft sind Verbindungen, die die INCI-Bezeichnung "Acrylates/$C_{10-30}$ Alkyl Acrylate Crosspolymer" tragen. Insbesondere vorteilhaft sind die unter den Handelsbezeichnungen Pemulen TR1 und Pemulen TR2 bei der B. F. Goodrich Company erhältlichen.

Vorteilhaft sind Verbindungen, die die INCI-Bezeichnung Ammoniumacryloyldimethyltaurate/Vinylpyrrolidoncopolymere tragen.

Erfindungsgemäß vorteilhaft weisen das oder die Ammoniumacryloyldimethyltaurate/Vinylpyrrolidoncopolymere die Summenformel $[C_7H_{16}N_2SO_4]_n$ $[C_6H_9NO]_m$ auf, einer statistischen Struktur wie folgt entsprechend

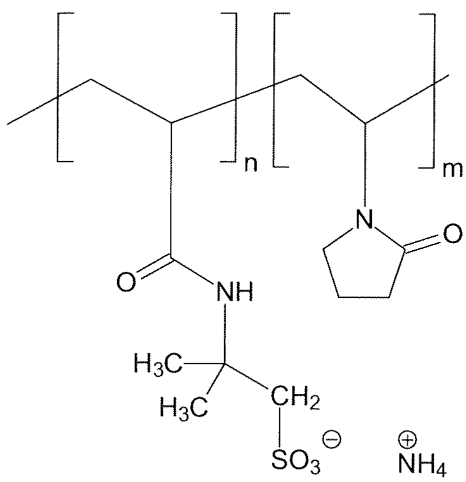

Bevorzugte Spezies im Sinne der vorliegenden Erfindung sind in den Chemical Abstracts unter den Registraturnummern 58374-69-9, 13162-05-5 und 88-12-0 abgelegt und erhältlich unter der Handelsbezeichnung Aristoflex® AVC der Gesellschaft Clariant GmbH.

Vorteilhaft sind ferner Copolymere/Crosspolymere umfassend Acryloyldimethyl Taurate, wie beispielsweise Simugel® EG oder Simugel® EG von der Gesellschaft Seppic S.A.

Weitere erfindungsgemäß vorteilhaft zu verwendende Hydrokolloide sind auch

1. in Wasser lösliche oder dispergierbare anionische Polyurethane, welche vorteilhaft erhältlich sind aus

   i) mindestens einer Verbindung, die zwei oder mehrere aktive Wasserstoffatome pro Moleküle enthält,
   ii) mindestens einem Säure- oder Salzgruppen enthaltenden Diol und
   iii) mindestens einem Diisocyanat.

Bei der Komponente i) handelt es sich insbesondere um Diole, Aminoalkohole, Diamine, Polyesterole, Polyetherole mit einem zahlenmittleren Molekulargewicht von jeweils bis zu 3000 oder deren Mischungen, wobei bis zu 3 Mol-% der genannten Verbindungen durch Triole oder Triamine ersetzt sein können. Bevorzugt sind Diole und Polyesterdiole. Insbesondere umfaßt die Komponente (a) mindestens 50 Gew.-%, bezogen auf das Gesamtgewicht der Komponente (a), eines Polyesterdiols. Als Polyesterdiole kommen alle diejenigen in Betracht, die üblicherweise zur Herstellung von Polyurethanen eingesetzt werden, insbesondere Umsetzungsprodukte aus Phthalsäure und Diethylenglycol, Isophthalsäure und 1,4-Butandiol, Isophthalsäure/Adipinsäure und 1,6-Hexandiol sowie Adipinsäure und Ethylenglycol oder 5-$NaSO_3$-Isophthalsäure, Phthalsäure, Adipinsäure und 1,6-Hexandiol.

Brauchbare Diole sind z.B. Ethylenglycol, Propylenglycol, Butylenglycol, Neopentylglycol, Polyetherole, wie Polyethylenglycole mit Molekulargewichten bis zu 3000, Blockcopolymerisate aus Ethylenoxid und Propylenoxid mit zahlenmittleren Molekulargewichten von bis zu 3000 oder Blockcopolymerisate aus Ethylenoxid, Propylenoxid und Butylenoxid, die die Alkylenoxideinheiten statistisch verteilt oder in Form von Blöcken einpolymerisiert enthalten. Bevorzugt sind Ethylenglycol, Neopentylglycol, Di-, Tri-, Tetra-, Penta oder Hexaethylenglyol. Brauchbare Diole sind außerdem Poly($\alpha$-hydroxycarbonsäure)diole.

Geeignete Aminoalkohole sind z.B. 2-Aminoethanol, 2-(N-Methylamino)ethanol, 3-Aminopropanol oder 4-Aminobutanol.

Geeignete Diamine sind z.B. Ethylendiamin, Propylendiamin, 1,4-Diaminobutan und 1,6-Diaminohexan sowie $\alpha,\omega$-Diamine, die durch Aminierung von Polyalkylenoxiden mit Ammoniak herstellbar sind.

Bei der Komponente ii) handelt es sich insbesondere um Dimethylolpropansäure oder Verbindungen der Formeln

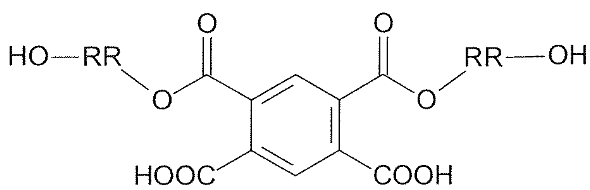

bzw.

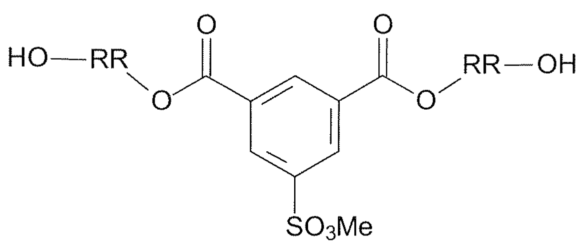

worin RR jeweils für eine $C_2$-$C_{18}$-Alkylengruppe steht und Me für Na oder K steht.

Bei der Komponente iii) handelt es sich insbesondere um Hexamethylendiisocyanat, Isophorondiisocyanat, Methyldiphenylisocyanat (MDI) und/oder Toluylendiisocyanat.

Die Polyurethane sind dadurch erhältlich, daß man die Verbindungen der Gruppen i) und ii) unter einer Inertgasatmosphäre in einem inerten Lösemittel bei Temperaturen von 70 bis 130°C mit den Verbindungen der Gruppe iii) umsetzt. Diese Umsetzung kann gegebenenfalls in Gegenwarte von Kettenverlängerern durchgeführt werden, um Polyurethane mit höheren Molekulargewichten herzustellen. Wie bei der Herstellung Polyurethanen üblich, werden die Komponenten [(i)+(ii)]:iii) vorteilhaft im molaren Verhältnis von 0,8 bis 1,1 : 1 eingesetzt. Die Säurezahl der Polyurethane wird von der Zusammensetzung und der Konzentration der Verbindungen der Komponente (ii) in der Mischung aus den Komponenten (i)+(ii) bestimmt.

Die Polyurethane haben K-Werte nach H. Fikentscher (bestimmt in 0,1 gew.-%igen Lösungen in N-Methylpyrrolidon bei 25 °C und pH 7) von 15 bis 100, vorzugsweise 25 bis 50.

Der auch als Eigenviskosität bezeichnete K-Wert ist ein über Viskositätsmessungen von Polymerlösungen einfach zu bestimmender und daher im techn. Bereich häufig benützter Parameter zur Charakterisierung von Polymeren. Für eine bestimmte Polymer-Sorte wird er unter standardisierten Meßbedingungen als alleine abhängig von der mittleren Molmasse der untersuchten Probe angenommen und über die Beziehung K-Wert = 1000 k nach der Fikentscher-Gleichung

$$k = \frac{1{,}5\lg\eta_r - 1 \pm \sqrt{1 + \left(\frac{2}{c} + 2 + 1{,}5\lg\eta_r\right) \cdot 1{,}5\lg\eta_r}}{150 + 300c}$$

berechnet, in der bedeuten: $\eta_r$ = relative Viskosität (dynamische Viskosität der Lösgung/dynamische Viskosität des Lösemittels) und c = Massenkonzentration an Polymer in der Lösung (in $g/cm^3$).

Die Säuregruppen enthaltenden Polyurethane sind nach Neutralisation (teilweise oder vollständig) wasserlöslich bzw. ohne Zuhilfenahme von Emulgatoren dispergierbar. In aller Regel weisen die Salze der Polyurethane eine bessere Wasserlöslichkeit oder Dispergierbarkeit in Wasser auf als die nicht neuralisierten Polyurethane. Als Base für die Neutralisation der Polyurethane können Alkalimetallbasen wie Natronlauge, Kalilauge, Soda, Natriumhydrogencarbonat, Kaliumcarbonat oder Kaliumhydrogencarbonat und Erdalkalimetallbasen wie Calciumhydroxid, Calciumoxid, Magnesiumhydroxid oder Magnesiumcarbonat sowie Ammoniak und Amine verwendet werden. Besonders haben sich zur Neutralisation der Säuregruppen enthaltenden Polyurethane 2-Amino-2-Methylpropanol, Diethylaminopropylamin und Triisopropanolamin bewährt. Die Neutralisation der Säuregruppen enthaltenden Polyurethane kann auch mit Hilfe von Mischungen mehrerer Basen vorgenommen werden, z.B. Mischungen aus Natronlauge und Triisopropanolamin. Die Neutralisation kann je nach Anwendungszweck partiell z.B. zu 20 bis 40 % oder vollständig, d.h. zu 100 % erfolgen.

Diese Polymere und ihre Herstellung sind in DE-A-42 25 045 näher beschrieben, auf die hiermit in vollem Umfang Bezug genommen wird.

2. In Wasser lösliche oder dispergierbare, kationische Polyurethane und Polyharnstoffe aus

a) mindestens einem Diisocyanat, welches bereits vorher mit einer oder mehreren Verbindungen, die zwei oder mehrere aktive Wasserstoffatome pro Moleküle enthalten, umgesetzt worden sein kann, und
b) mindestens einem Diol, primären oder sekundären Aminoalkohol, primären oder sekundärem Diamin oder primären oder sekundären Triamin mit einem oder mehreren tertiären, -quaternären oder protonierten tertiären Aminostickstoffatomen.

Bevorzugte Diisocyanate sind wie oben unter 1) angegeben. Verbindungen mit zwei oder mehreren aktiven Wasserstoffatomen sind Diole, Aminoalkohole, Diamine, Polyesterole. Polyamiddiamine und Polyetherole. Geeignete Verbindungen dieser Art sind wie oben unter 1) angegeben.

Die Herstellung der Polyurethane erfolgt wie oben unter 1) beschrieben. Geladene kationische Gruppierungen lassen sich aus den vorliegenden tertiären Aminostickstoffatomen entweder durch Protonierung, z.B. mit Carbonsäuren wir Milchsäure, oder durch Quaternisierung, z.B. mit Alkylierungsmitteln wie $C_1$-bis $C_4$-Alkylhalogeniden oder -sulfaten in den Polyharnstoffen erzeugen. Beispiele solcher Alkylierungsmittel sind Ethylchlorid, Ethylbromid, Methylchlorid, Methylbromid, Dimethylsulfat und Diethylsulfat.

Diese Polymere und ihre Herstellung sind in der DE-A-42 41 118 näher beschrieben, auf die hiermit in vollem Umfang Bezug genommen wird.

3. Lineare Polyurethane mit Carboxylatgruppen aus

i) einer 2,2-Hydroxymethyl-substituierten Carbonsäure der Formel

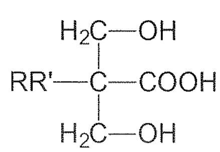

worin RR' für ein Wasserstoffatom oder eine $C_1$-$C_{20}$-Alkylgruppe steht, die in einer Menge verwendet wird, welche ausreicht, daß in dem Polyurethan 0,35 bis 2,25 Milliäquivalente Carboxylgruppen pro g Polyurethan vorhanden sind,

ii) 10 bis 90 Gew.-%, bezogen auf das Gewicht des Polyurethans, einer oder mehrerer organischer Verbindungen mit nicht mehr als zwei aktiven Wasserstoffatomen und

iii) einem oder mehreren organischen Diisocyanaten.

Die im Polyurethan enthaltenden Carboxylgruppen werden abschließend mit einer geeigneten Base zumindest teilweise neutralisiert. Diese Polymere und ihre Herstellung sind in der EP-A-619 111 beschrieben, auf die hiermit in vollem Umfang Bezug genommen wird.

4. Carboxylhaltige Polykondensationsprodukte aus Anhydriden von Tri- oder Tetracarbonsäuren und Diolen, Diaminen oder Aminoalkoholen (Polyester, Polyamide oder Polyesteramide). Diese Polymere und ihre Herstellung sind in der DE-A-42 24 761 näher beschrieben, auf die hiermit in vollem Umfang Bezug genommen wird.

5. Polyacrylate und Polymethacrylate, wie sie in den DE-A-43 14 305, 36 27 970 und 29 17 504 näher beschrieben sind. Auf diese Publikationen wird hiermit in vollem Umfang Bezug genommen.

Die erfindungsgemäß zur Anwendung kommenden Polymere besitzen vorzugsweise einen K-Wert von 25 bis 100, bevorzugt 25 bis 50. Die Polymere sind in dem erfindungsgemäßen Mittel im allgemeinen in einer Menge im Bereich von 0,2 bis 20 Gew.-%, bezogen auf das Gesamtgewicht des Mittels, enthalten. Das Salz kommt in einer zur Verbesserung der Austauschbarkeit der Polymeren wirksamen Menge zur Anwendung. Im allgemeinen setzt man das Salz in einer Menge von 0,02 bis 10 Gew.-%, vorzugsweise 0,05 bis 5 Gew.-% und insbesondere 0,1 bis 3 Gew.-%, bezogen auf das Gesamtgewicht des Mittels, ein.
Die Gesamtmenge an einem oder mehreren Hydrokolloiden wird in den fertigen kosmetischen oder dermatologischen Zubereitungen vorteilhaft kleiner als 5% Gew. %, bevorzugt zwischen 0,1 und 1,0 Gew.-%, bezogen auf das Gesamtgewicht der Zubereitungen, gewählt.
Ferner kann es von Vorteil sein, Zubereitungen gemäß der Erfindung grenz- bzw. oberflächenaktive Agentien zuzufügen, beispielsweise kationische Emulgatoren wie insbesondere quaternäre Tenside.
Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder - bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.
Vorteilhaft ist auch, kationische Polymere (z.B. Jaguar® C 162 [Hydroxypropyl Guar Hydroxypropyltrimonium Chloride] bzw. modifizierten Magnesiumaluminiumsilikaten (z.B. Quaternium-18-Hectorit, welches z. B. unter der Handelsbezeichnung Bentone® 38 bei der Firma Rheox erhältlich ist, oder Stearalkonium Hectorit, welches z. B. unter der Handelsbezeichnung Softisan® Gel bei der Hüls AG erhältlich ist), einzusetzen.
Erfindungsgemäße Zubereitungen können vorteilhaft auch Ölverdickungsmittel enthalten, um die taktilen Eigenschaften der Emulsion und die Stiftkonsistenz zu verbessern. Vorteilhafte Ölverdickungsmittel im Sinne der vorliegenden Erfindung sind beispielsweise weitere Feststoffe, wie z. B. hydrophobe Siliciumoxide des Typs Aerosil®, welche von der Degussa AG erhältlich sind. Vorteilhafte Aerosil®-Typen sind beispielsweise Aerosil® OX50, Aerosil® 130, Aerosil® 150, Aerosil® 200, Aerosil® 300, Aerosil® 380, Aerosil® MOX 80, Aerosil® MOX 170, Aerosil® COK 84, Aerosil® R 202, Aerosil® R 805, Aerosil® R 812, Aerosil® R 972, Aerosil® R 974 und/oder Aerosil® R976.
Ferner sind auch sogenannte Metallseifen (d. h. die Salze höherer Fettsäuren mit Ausnahme der Alkalisalze) vorteilhafte Ölverdickungsmittel im Sinne der vorliegenden Erfindung, wie beispielsweise Aluminium-Stearat, Zink-Stearat und/oder MagnesiumStearat.
Ebenfalls vorteilhaft ist, Zubereitungen gemäß der Erfindung amphotere bzw. zwitterionische Tenside (z.B. Cocoamidopropylbetain) und Moisturizer (z.B. Betain) zuzusetzen. Vorteilhaft zu verwendende amphotere Tenside sind beispielsweise Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat, N-Alkylaminosäuren, beispielsweise Aminopropylalkylglutamid, Alkylaminopropionsäure, Natriumalkylimidodipropionat und Lauroamphocarboxyglycinat.
Die Menge der ober- bzw. grenzflächenaktiven Substanzen (eine oder mehrere Verbindungen) in den Zubereitungen gemäß der Erfindung beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Eine erstaunliche Eigenschaft der erfindungsgemäße Zubereitungen ist, daß diese sehr gute Vehikel für kosmetische oder dermatologische Wirkstoffe in die Haut sind, wobei bevorzugte Wirkstoffe die vorab erwähnten Antioxidantien sind, welche die Haut vor oxidativer Beanspruchung schützen können.

Erfindungsgemäß können die Wirkstoffe (eine oder mehrere Verbindungen) auch sehr vorteilhaft gewählt werden aus der Gruppe der lipophilen Wirkstoffe, insbesondere aus folgender Gruppe:

Acetylsalicylsäure, Atropin, Azulen, Hydrocortison und dessen Derivaten, z. B. Hydrocortison-17-valerat, Vitamine der B- und D-Reihe, sehr günstig das Vitamin $B_1$, Vitamin $B_5$, das Vitamin $B_{12}$ das Vitamin $D_1$, aber auch Bisabolol, ungesättigte Fettsäuren, namentlich die essentiellen Fettsäuren (oft auch Vitamin F genannt), insbesondere die γ-Linolensäure, Ölsäure, Eicosapentaensäure, Docosahexaensäure und deren Derivate, Chloramphenicol, Coffein, Prostaglandine, Dioic Acid, Allantoin, Harnstoff, Thymol, Campher, Extrakte oder andere Produkte pflanzlicher und tierischer Herkunft, z. B. Nachtkerzenöl, Borretschöl oder Johannisbeerkernöl, Fischöle, Lebertran aber auch Ceramide und ceramidähnliche Verbindungen und so weiter.

Vorteilhaft ist es auch, die Wirkstoffe aus der Gruppe der rückfettenden Substanzen zu wählen, beispielsweise Purcellinöl, Eucerit® und Neocerit®.

Besonders vorteilhaft werden der oder die Wirkstoffe ferner gewählt aus der Gruppe der NO-Synthasehemmer, insbesondere wenn die erfindungsgemäßen Zubereitungen zur Behandlung und Prophylaxe der Symptome der intrinsischen und/oder extrinsischen Hautalterung sowie zur Behandlung und Prophylaxe der schädlichen Auswirkungen ultravioletter Strahlung auf die Haut dienen sollen.

Bevorzugter NO-Synthasehemmer ist das Nitroarginin.

Weiter vorteilhaft werden der oder die Wirkstoffe gewählt aus der Gruppe, welche Catechine und Gallensäureester von Catechinen und wäßrige bzw. organische Extrakte aus Pflanzen oder Pflanzenteilen umfaßt, die einen Gehalt an Catechinen oder Gallensäureestern von Catechinen aufweisen, wie beispielsweise den Blättern der Pflanzenfamilie Theaceae, insbesondere der Spezies Camellia sinensis (grüner Tee). Insbesondere vorteilhaft sind deren typische Inhaltsstoffe (wie z. B. Polyphenole bzw. Catechine, Coffein, Vitamine, Zucker, Mineralien, Aminosäuren, Lipide).

Catechine stellen eine Gruppe von Verbindungen dar, die als hydrierte Flavone oder Anthocyanidine aufzufassen sind und Derivate des "Catechins" (Catechol, 3,3',4',5,7-Flavanpentaol, 2-(3,4-Dihydroxyphenyl)-chroman-3,5,7-triol) darstellen. Auch Epicatechin ((2R,3R)-3,3',4',5,7-Flavanpentaol) ist ein vorteilhafter Wirkstoff im Sinne der vorliegenden Erfindung.

Vorteilhaft sind ferner pflanzliche Auszüge mit einem Gehalt an Catechinen, insbesondere Extrakte des grünen Tees, wie z. B. Extrakte aus Blättern der Pflanzen der Spezies Camellia spec., ganz besonders der Teesorten Camellia sinenis, C. assamica, C. taliensis bzw. C. irrawadiensis und Kreuzungen aus diesen mit beispielsweise Camellia japonica.

Bevorzugte Wirkstoffe sind ferner Polyphenole bzw. Catechine aus der Gruppe (-)-Catechin, (+)-Catechin, (-)-Catechingallat, (-)-Gallocatechingallat, (+)-Epicatechin, (-)-Epicatechin, (-)-Epicatechin Gallat, (-)-Epigallocatechin, (-)-Epigallocatechingallat.

Auch Flavon und seine Derivate (oft auch kollektiv "Flavone" genannt) sind vorteilhafte Wirkstoffe im Sinne der vorliegenden Erfindung. Sie sind durch folgende Grundstruktur gekennzeichnet (Substitutionspostitionen angegeben):

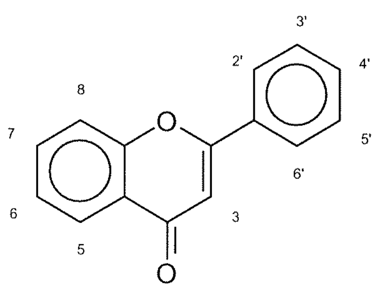

Einige der wichtigeren Flavone, welche auch bevorzugt in erfindungsgemäßen Zubereitungen eingesetzt werden können, sind in der nachstehenden Tabelle 2 aufgeführt:

Tabelle 2

| OH-Substitutionspositionen | | | | | | | | |
|---|---|---|---|---|---|---|---|---|
| | 3 | 5 | 7 | 8 | 2' | 3' | 4' | 5' |
| Flavon | - | - | - | - | - | - | - | - |
| Flavonol | + | - | - | - | - | - | - | - |
| Chrysin | - | + | + | - | - | - | - | - |
| Galangin | + | + | + | - | - | - | - | - |
| Apigenin | - | + | + | - | - | - | + | - |
| Fisetin | + | - | + | - | - | + | + | - |
| Luteolin | - | + | + | - | - | + | + | - |
| Kämpferol | + | + | + | - | - | - | + | - |
| Quercetin | + | + | + | - | - | + | + | - |
| Morin | + | + | + | - | + | - | + | - |
| Robinetin | + | - | + | - | - | + | + | + |
| Gossypetin | + | + | + | + | - | + | + | - |
| Myricetin | + | + | + | - | - | + | + | + |

In der Natur kommen Flavone in der Regel in glycosidierter Form vor.

Erfindungsgemäß werden die Flavonoide bevorzugt gewählt gewählt aus der Gruppe der Substanzen der generischen Strukturformel

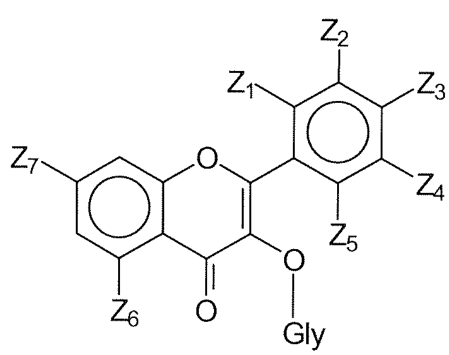

,

wobei $Z_1$ bis $Z_7$ unabhängig voneinander gewählt werden aus der Gruppe H, OH, Alkoxy- sowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Erfindungsgemäß können die Flavonoide aber auch vorteilhaft gewählt werden aus der Gruppe der Substanzen der generischen Strukturformel

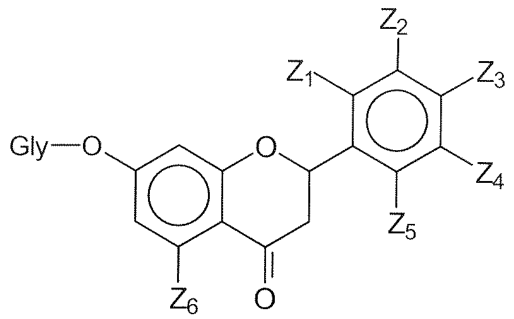

,

wobei $Z_1$ bis $Z_6$ unabhängig voneinander gewählt werden aus der Gruppe H, OH, Alkoxy- sowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Bevorzugt können solche Strukturen gewählt werden aus der Gruppe der Substanzen der generischen Strukturformel

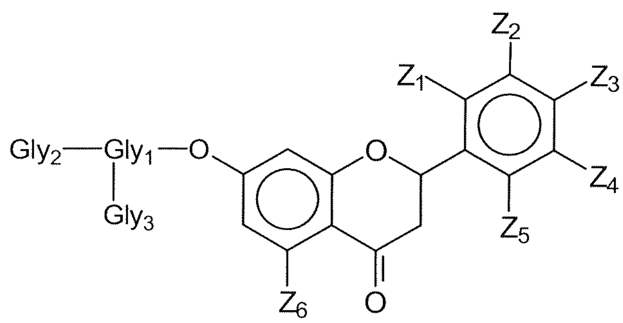

wobei $Gly_1$, $Gly_2$ und $Gly_3$ unabhängig voneinander Monoglycosidreste oder darstellen. $Gly_2$ bzw. $Gly_3$ können auch einzeln oder gemeinsam Absättigungen durch Wasserstoffatome darstellen.

Bevorzugt werden $Gly_1$, $Gly_2$ und $Gly_3$ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Vorteilhaft werden $Z_1$ bis $Z_5$ unabhängig voneinander gewählt aus der Gruppe H, OH, Methoxy-, Ethoxy- sowie 2-Hydroxyethoxy-, und die Flavonglycoside haben die Struktur

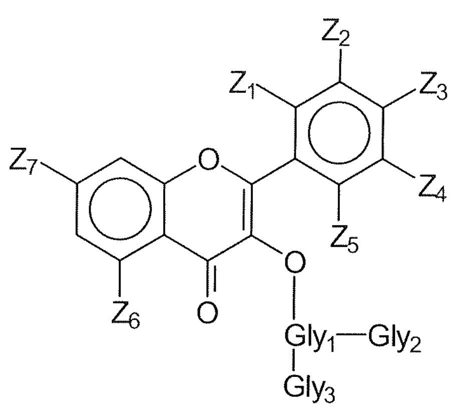

Besonders vorteilhaft werden die erfindungsgemäßen Flavonglycoside aus der Gruppe, welche durch die

folgende Struktur wiedergegeben werden:

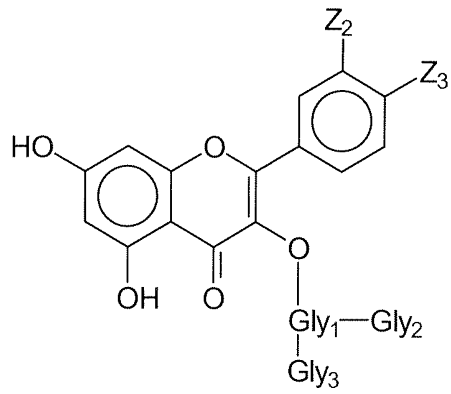

wobei $Gly_1$, $Gly_2$ und $Gly_3$ unabhängig voneinander Monoglycosidreste oder darstellen. $Gly_2$ bzw. $Gly_3$ können auch einzeln oder gemeinsam Absättigungen durch Wasserstoffatome darstellen.

Bevorzugt werden $Gly_1$, $Gly_2$ und $Gly_3$ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Besonders vorteilhaft im Sinne der vorliegenden Erfindung ist, das oder die Flavonglycoside zu wählen aus der Gruppe α-Glucosylrutin, α-Glucosylmyricetin, α-Glucosylisoquercitrin, α-Glucosylisoquercetin und α-Glucosylquercitrin.

Erfindungsgemäß besonders bevorzugt ist α-Glucosylrutin.

Erfindungsgemäß vorteilhaft sind auch Naringin (Aurantiin, Naringenin-7-rhamnoglucosid), Hesperidin (3',5,7-Trihydroxy-4'-methoxyflavanon-7-rutinosid, Hesperidosid, Hesperetin-7-O-rutinosid). Rutin (3,3',4',5,7-Pentahydroxyflyvon-3-rutinosid, Quercetin-3-rutinosid, Sophorin, Birutan, Rutabion, Taurutin, Phytomelin, Melin), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Dihydrorobinetin (3,3',4',5',7-Pentahydroxyflavanon), Taxifolin (3,3',4',5,7-Pentahydroxyflavanon), Eriodictyol-7-glucosid (3',4',5,7-Tetrahydroxyflavanon-7-glucosid), Flavanomarein (3',4',7,8-Tetrahydroxyflavanon-7-glucosid) und Isoquercetin (3,3',4',5,7-Pentahydroxyflavanon-3-(β-D-Glucopyranosid).

Vorteilhaft ist es auch, dem oder die Wirkstoffe aus der Gruppe der Ubichinone und Plastochinone zu wählen.

Ubichinone zeichnen sich durch die Strukturformel

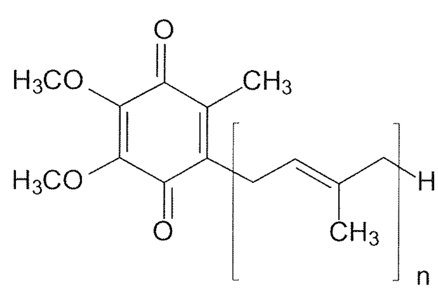

aus und stellen die am weitesten verbreiteten und damit am besten untersuchten Biochinone dar. Ubichinone werden je nach Zahl der in der Seitenkette verknüpften Isopren-Einheiten als Q-1, Q-2, Q-3 usw. oder nach Anzahl der C-Atome als U-5, U-10, U-15 usw. bezeichnet. Sie treten bevorzugt mit bestimmten Kettenlängen auf, z. B. in einigen Mikroorganismen und Hefen mit n=6. Bei den meisten Säugetieren einschließlich des Menschen überwiegt Q10.

Besonders vorteilhaft ist Coenzym Q10, welches durch folgende Strukturformel gekennzeichnet ist:

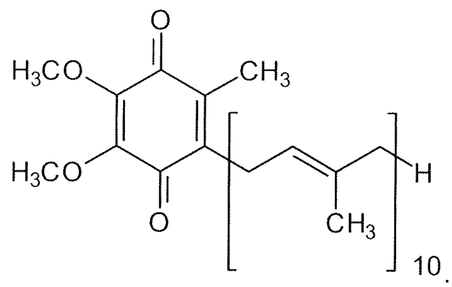

Plastochinone weisen die allgemeine Strukturformel

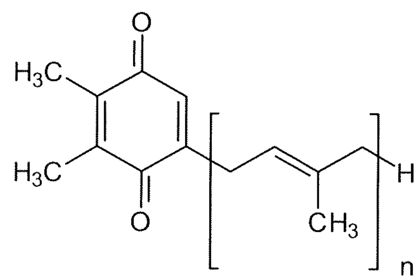

auf. Plastoschinone unterscheiden sich in der Anzahl n der Isopren-Reste und werden endsprechend bezeichnet, z. B. PQ-9 (n=9). Ferner existieren andere Plastochinone mit unterschiedlichen Substituenten am Chinon-Ring.

Auch Kreatin und/oder Kreatinderivate sind bevorzugte Wirkstoffe im Sinne der vorliegenden Erfindung. Kreatin zeichnet sich durch folgende Struktur aus:

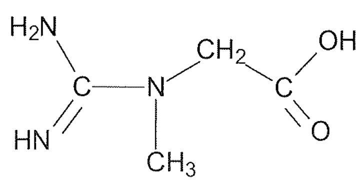

Bevorzugte Derivate sind Kreatinphosphat sowie Kreatinsulfat, Kreatinacetat, Kreatinascorbat und die an der Carboxylgruppe mit mono- oder polyfunktionalen Alkoholen veresterten Derivate.

Ein weiterer vorteilhafter Wirkstoff ist Isoflavon 150 [3-Hydroxy-4-(trimethylammonio)-buttersäurebetain]. Auch Acylsoflavon 150e, welche gewählt aus der Gruppe der Substanzen der folgenden allgemeinen Strukturformel

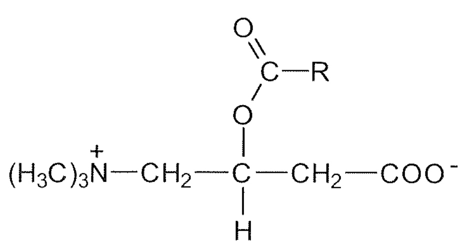

,

wobei R gewählt wird aus der Gruppe der verzweigten und unverzweigten Alkylreste mit bis zu 10 Kohlenstoffatomen sind vorteilhafte Wirkstoffe im Sinne der vorliegenden Erfindung. Bevorzugt sind Propionylcarnitin und insbesondere Acetylcarnitin. Beide Entantiomere (D- und L-Form) sind vorteilhaft im Sinne der vorliegenden Erfindung zu verwenden. Es kann auch von Vorteil sein, beliebige Enantiomerengemische, beispielsweise ein Racemat aus D- und L-Form, zu verwenden.

Weitere vorteilhafte Wirkstoffe sind Sericosid, Pyridoxol, Vitamin K, Biotin und Aromastoffe.

Die Liste der genannten Wirkstoffe bzw. Wirkstoffkombinationen, die in den erfindungsgemäßen Zubereitungen verwendet werden können, soll selbstverständlich nicht limitierend sein. Die Wirkstoffe können einzelnen oder in beliebigen Kombinationen miteinander verwendet werden.

Die Menge solcher Wirkstoffe (eine oder mehrere Verbindungen) in den Zubereitungen gemäß der Erfindung beträgt vorzugsweise 0,001 bis 10 Gew.-%, besonders bevorzugt 0,05 - 5 Gew.-%, insbesondere 0,1 - 3 Gew.-%, bezogen auf das Gesamtgewicht der Zubereitung.

Es kann auch gegebenenfalls vorteilhaft im Sinne der vorliegenden Erfindung sein, den Zubereitungen gemäß der Erfindung Farbstoffe und/oder -pigmente einzuverleiben.

Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. $Fe_2O_3$, $Fe_3O_4$, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem Rowe Colour Index, 3. Auflage, Society of Dyers and Colourists, Bradford, England, 1971 entnommen.

| Chemische oder sonstige Bezeichnung | CIN | Farbe |
|---|---|---|
| Pigment Green | 10006 | grün |
| Acid Green 1 | 10020 | grün |
| 2,4-Dinitrohydroxynaphthalin-7-sulfosäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |
| 4-(2'-Methoxy-5'-sulfosäurediethylamid-1'-phenylazo)-3-hydroxy-5"-chloro-2",4"-dimethoxy-2-naphthoesäureanilid | 12490 | rot |
| Disperse Yellow 16 | 12700 | gelb |
| 1-(4-Sulfo-1-phenylazo)-4-amino-benzol-5-sulfosäure | 13015 | gelb |
| 2,4-Dihydroxy-azobenzol-4'-sulfosäure | 14270 | orange |
| 2-(2,4-Dimethylphenylazo-5-suffosäure)-1-hydroxynaphthalin-4-sulfosäure | 14700 | rot |
| 2-(4-Sulfo-1-naphthylazo)-1-naphthol-4-sulfosäure | 14720 | rot |
| 2-(6-Sulfo-2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2-hydroxynaphthalin | 15525 | rot |
| 1-(3-Methyl-phenylazo-4-sulfosäure)-2-hydroxynaphthalin | 15580 | rot |
| 1-(4',(8')-Sulfosäurenaphthylazo)-2-hydroxynaphthalin | 15620 | rot |
| 2-Hydroxy-1,2'-azonaphthalin-1'-sulfosäure | 15630 | rot |
| 3-Hydroxy-4-phenylazo-2-naphthylcarbonsäure | 15800 | rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxy-naphthalin-3-carbonsäure | 15865 | rot |
| 1-(2-Sulfo-1-naphthylazo)-2-hydroxynaphthalin-3-carbonsäure | 15880 | rot |
| 1-(3-Sulfo-1-phenylazo)-2-naphthol-6-sulfosäure | 15980 | orange |
| 1-(4-Sulfo-1-phenylazo)-2-naphthol-6-sulfosäure | 15985 | gelb |
| Allura Red | 16035 | rot |
| 1-(4-Sulfo-1-naphthylazo)-2-naphthol-3,6-disulfosäure | 16185 | rot |

(fortgesetzt)

| Chemische oder sonstige Bezeichnung | CIN | Farbe |
|---|---|---|
| Acid Orange 10 | 16230 | orange |
| 1-(4-Sulfo-1-naphthylazo)-2-naphthol-6,8-disulfosäure | 16255 | rot |
| 1-(4-Sulfo-1-naphthylazo)-2-naphthol-3,6,8-trisulfosäure | 16290 | rot |
| 8-Amino-2 -phenylazo- 1 -naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxy-pyrazolon-3-carbonsäure | 19140 | gelb |
| Pigment Yellow 16 | 20040 | gelb |
| 2,6-(4'-Sulfo-2", 4"-dimethyl)-bis-phenylazo)1,3-dihydroxybenzol | 20170 | orange |
| Acid Black 1 | 20470 | schwarz |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"-Sulfo-1 "-phenylazo)-7'-sulfo-1'-naphthylazo]-1-hydroxy-7-aminonaphthalin-3,6-disulfosäure | 27755 | schwarz |
| 4'-[(4"-Sulfo-1"-phenylazo)-7'-sulfo-1'-naphthylazo]-1-hydroxy-8-acetyl-aminonaphthalin-3,5-disulfosäure | 28440 | schwarz |
| Direct Orange 34, 39, 44, 46, 60 | 40215 | orange |
| Food Yellow | 40800 | orange |
| trans-β-Apo-8'-Carotinaldehyd ($C_{30}$) | 40820 | orange |
| trans-Apo-8'-Carotinsäure ($C_{30}$)-ethylester | 40825 | orange |
| Canthaxanthin | 40850 | orange |
| Acid Blue 1 | 42045 | blau |
| 2,4-Disulfo-5-hydroxy-4'-4"-bis-(diethylamino)triphenyl-carbinol | 42051 | blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | grün |
| Acid Blue 7 | 42080 | blau |
| (N-Ethyl-p-sulfobenzyl-amino)-phenyl-(2-sulfophenyl)-methylen-(N -ethyl-N-p-su Ifo-benzyl)L1$^{2,s}$ -cyclohexad ien im in | 42090 | blau |
| Acid Green 9 | 42100 | grün |
| Diethyl-di-sulfobenzyl-di-4-amino-2-chlor-di-2-methyl-fuchsonimmonium | 42170 | grün |
| Basic Violet 14 | 42510 | violett |
| Basic Violet 2 | 42520 | violett |
| 2'-Methyl-4'-(N-ethyl-N-m-sulfobenzyl)-amino-4"-(N-diethyl)-amino-2-methyl-N-ethylN-m-sulfobenzyl-fuchsonimmonium | 42735 | blau |
| 4'-(N-Dimethyl)-amino-4"-(N-phenyl)-aminonaphtho-N-dimethyl-fuchsonimmonium | 44045 | blau |
| 2-Hydroxy-3,6-disulfo-4,4'-bis-dimethylamino-naphthofuchsonimmonium | 44090 | grün |
| Acid Red 52 | 45100 | rot |
| 3-(2'-Methylphenylamino)-6-(2'-methyl-4'-sulfophenylamino)-9-(2"-carboxyphenyl)-xantheniumsalz | 45190 | violett |
| Acid Red 50 | 45220 | rot |
| Phenyl-2-oxyfluoron-2-carbonsäure | 45350 | gelb |

(fortgesetzt)

| Chemische oder sonstige Bezeichnung | CIN | Farbe |
|---|---|---|
| 4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |
| 3',4',5',6'-Tetrachlor-2,4,5,7-tetrabromfluorescein | 45410 | rot |
| 4,5-Diiodfluorescein | 45425 | rot |
| 2,4,5,7-Tetraiodfluorescein | 45430 | rot |
| Chinophthalon | 47000 | gelb |
| Chinophthalon-disulfosäure | 47005 | gelb |
| Acid Violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4-N-phenyl-aminoanthrachinon | 60724 | violett |
| 1-Hydroxy-4-(4'-methylphenylamino)-anthrachinon | 60725 | violett |
| Acid Violet 23 | 60730 | violett |
| 1,4-Di(4'-methyl-phenylamino)-anthrachinon | 61565 | grün |
| 1,4-Bis-(o-sulfo-p-toluidino)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,7'-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| Chlorierte Phthalocyanine | 74260 | grün |
| Natural Yellow 6,19; Natural Red 1 | 75100 | gelb |
| Bixin, Nor-Bixin | 75120 | orange |
| Lycopin | 75125 | gelb |
| trans-alpha-, beta- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na, Al, Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | rot |
| Bariumsulfat | 77120 | weiß |

(fortgesetzt)

| Chemische oder sonstige Bezeichnung | CIN | Farbe |
|---|---|---|
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268:1 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Mangananimoniumdiphosphat | 77742 | violett |
| Manganphosphat; $Mn_3(PO_4)_2 \cdot 7$ H20 | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyryliumsalze, Anthocyane | | rot |
| Aluminium-, Zink-, Magnesium- und Calciumstearat | | weiß |
| Bromthymolblau | | blau |
| Bromkresolgrün | | grün |
| Acid Red 195 | | rot |

Es kann ferner günstig sein, als Farbstoff eine oder mehrer Substanzen aus der folgenden Gruppe zu wählen: 2,4-Dihydroxyazobenzol, 1-(2'-Chlor-4'-nitro-1'-phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77 492), Manganammoniumdiphosphat und Titandioxid.

Ferner vorteilhaft sind öllösliche Naturfarbstoffe, wie z. B. Paprikaextrakte, β-Carotin oder Cochenille.

Vorteilhaft im Sinne der vorliegenden Erfindung sind ferner Gelcrèmes mit einem Gehalt an Perlglanzpigmenten. Bevorzugt sind insbesondere die im folgenden aufgelisteten Arten von Perlglanzpigmenten:

Natürliche Perlglanzpigmente, wie z. B.

- "Fischsilber" (Guanin/Hypoxanthin-Mischkristalle aus Fischschuppen) und

- "Perlmutt" (vermahlene Muschelschalen)

Monokristalline Perlglanzpigmente wie z. B. Bismuthoxychlorid (BiOCl)
Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteihaft ist z. B. das unter der CIN 77163 aufgelistete Glanzpigment.
Vorteilhaft sind ferner beispielsweise die folgenden Perlglanzpigmentarten auf Basis von Glimmer/Metalloxid:

| Gruppe | Belegung / Schichtdicke | Farbe |
|---|---|---|
| Silberweiße Perlglanzpigmente | $TiO_2$: 40 - 60 nm | silber |
| Interferenzpigmente | $TiO_2$: 60 - 80 nm | gelb |
| | $TiO_2$: 80 - 100 nm | rot |
| | $TiO_2$: 100 - 140 nm | blau |
| | $TiO_2$: 120 - 160 nm | grün |
| Farbglanzpigmente | $Fe_2O_3$ | bronze |
| | $Fe_2O_3$ | kupfer |
| | $Fe_2O_3$ | rot |
| | $Fe_2O_3$ | rotviolett |
| | $Fe_2O_3$ | rotgrün |
| | $Fe_2O_3$ | schwarz |
| Kombinationspigmente | $TiO_2$ / $Fe_2O_3$ | Goldtöne |
| | $TiO_2$ / $Cr_2O_3$ | grün |
| | $TiO_2$ / Berliner Blau | tiefblau |
| | $TiO_2$ / Carmin | rot |

Besonders bevorzugt sind z.B. die von der Firma Merck unter den Handelsnamen Timiron, Colorona oder Dichrona erhältlichen Perlglanzpigmente.
Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit $TiO_2$ und $Fe_2O_3$ beschichtete $SiO_2$-Partikel ("Ronaspheren"), die von der Firma Merck vertrieben werden und sich besonders für die optische Reduktion feiner Fältchen eignen.
Es kann darüber hinaus von Vorteil sein, gänzlich auf ein Substrat wie Glimmer zu verzichten. Besonders bevorzugt sind Perlglanzpigmente, welche unter der Verwendung von $SiO_2$ hergestellt werden. Solche Pigmente, die auch zusätzlich gonichromatische Effekte haben können, sind z. B. unter dem Handelsnamen Sicopearl Fantastico bei der Firma BASF erhältlich.
Weiterhin vorteilhaft können Pigmente der Firma Engelhard / Mearl auf Basis von Calcium Natrium Borosilikat, die mit Titandioxid beschichtet sind, eingesetzt werden. Diese sind unter dem Namen Reflecks erhältlich. Sie weisen durch ihrer Partikelgröße von 40 - 180 µm zusätzlich zu der Farbe einen Glitzereffekt auf.
Besonders vorteilhaft sind ferner auch Effektpigmente, welche unter der Handelsbezeichnung Metasomes Standard / Glitter in verschiedenen Farben (yellow, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (CI) Nummern 19140, 77007, 77289, 77491) vor.
Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 15 Gew.-%, insbesondere von 1,0 bis 10 Gew.-% gewählt, jeweils bezogen auf das Gesamtgewicht der Zubereitungen.

Die Herstellung erfindungsgemäßer Zubereitungen erfolgt unter den dem Fachmanne geläufigen Bedingungen. Es werden in der Regel die Bestandteile der Ölphase bzw. der Wasserphase gesondert zusammengegeben und erwärmt, und sodann unter Rühren und, besonders vorteilhaft, unter Homogenisieren, ganz besonders vorteilhaft unter Rühren mit mittlerem bis hohen Energieeintrag, vorteilhaft mit Hilfe einer Zahnkranzdispergiermaschine mit einer Umdrehungszahl bis maximal 10000 U/min, vorzugsweise von 2500 bis 7700 U/min, miteinander vereinigt.

Die nachfolgenden Beispiele sollen die vorliegende Erfindung verdeutlichen, aber nicht einschränken. Die Zahlenangaben beziehen sich auf Gew.-%, sofern nichts Anderes angegeben ist.

**Beispiele 1-10: O/W-Cremes**

| Beispiel Nummer | 1 | 2 | 3 | 4 | 5 |
|---|---|---|---|---|---|
| Glycerylstearatcitrat | 2 | | | | |
| Glycerylstearat, selbstemulgierend | | 5 | 3 | 1 | 2 |
| PEG-40-Stearat | | | 1 | | 1 |
| Stearinsäure | | | | 4 | |
| Myristylmyristat | 1 | | | | 1 |
| Behenylalkohol | | | | 1 | |
| Stearylalkohol | 2 | 1 | | | |
| Cetearylalkohol | | | | | 2 |
| Cetylalkohol | 1 | | 3 | 3 | |
| Hydrierte Kokosfettglyceride (Hydrogenated Coco Glycerides) | 2 | | | | |
| Sheabutter | | 1 | | | 2 |
| C12-15 Alkylbenzoat | | 3 | 2 | | 3 |
| Butylenglycol Dicaprylat/Dicaprat | 1 | | | 1 | |
| Caprylsäure/Caprinsäure Triglycerid | | 1 | 2 | 2 | 2 |
| Ethylhexylkokosfettsäureester | 3 | | | | 1 |
| Octyldodecanol | | | 1 | | |
| Jojobaöl | | | | 1 | |
| Mineralöl | | 1 | | | |
| Vaseline | 1 | | 1 | | 2 |
| Cyclomethicon | 3 | 2 | 4 | 3 | 5 |
| Dimethicon | | | 1 | 1 | |
| Dicaprylylether | 1 | 3 | 2 | | |
| Dicarprylylcarbonat | | | | 3 | |
| $TiO_2$ | 1 | | 1 | | 1 |
| Ethylhexylmethoxycinnamat | 5 | 3 | 5 | | 3 |
| Ethylhexyltriazon | | 1 | | | |
| Ethylhexylcyanodiphenyl-acrylat (Octocrylen) | | | | 5 | 3 |
| Butylmethoxydibenzoylmethan | 1 | | | | |
| Bis-Ethylhexyloxyphenolmethoxyphenyltriazine | | 1 | | | |
| Ethylhexylsalicylat | 1 | | | | |

(fortgesetzt)

| Beispiel Nummer | 1 | 2 | 3 | 4 | 5 |
|---|---|---|---|---|---|
| 2-Hydroxy-4-Methoxy- benzophenon (Oxybenzone) | | | 2 | 3 | 2 |
| Phenylbenzimidazol sulfonsäure | | | | 2 | |
| Ubichinon (Q10) | | 0,01 | | | |
| Taurin | 1 | 2 | 0,1 | 0,5 | 0,3 |
| Sorbinsäure | 0,1 | 0,05 | | | |
| Tocopherylacetat | | | 1 | | 0,3 |
| Retinylpalmitat | 0,1 | | | | |
| Citronensäure, Natriumsalz | | 0.1 | | | |
| Natriumascorbylphosphat | | | | | 0,1 |
| Phenoxyethanol | 0,3 | | 0,3 | 0,2 | 0,4 |
| p-Hydroxybenzoesäuremethylester (Methylparaben) | 0,2 | | 0,1 | 0,3 | |
| p-Hydroxybenzoesäureethylester (Ethylparaben) | 0,1 | | | | 0,2 |
| p-Hydroxybenzoesäurepropylester (Propylparaben) | | | 0,1 | | |
| Hexamidindiisethionat | | 0,04 | | | |
| Diazolidinylharnstoff | | | | 0,2 | |
| 1,3-Dimethylol-5,5-dimethylhydantoin(DMDM Hydantoin) | | 0,2 | | | |
| lodopropynylbutylcarbamat | | | | | 0,05 |
| Ethanol denaturiert | 3 | 2 | | | |
| Xanthan Gummi | | | 0,1 | | |
| Polyacrylsäure (Carbomer) | 0,05 | | | | 0,1 |
| Ammoniumpolyacryloyldimethyltaurate | 0,4 | | | 0,3 | |
| Ammoniumacryloyldimethyltaurate/ Vinylpyrrolidoncopolymere | | 0,5 | 0,3 | | |
| Aluminium Stärke Octenylsuccinate | | | | | 0,5 |
| Glycerin | 10 | 6 | 7 | 6 | 5 |
| Dipropylenglycol | | | | | 2 |
| Butylenglycol | | 3 | | 3 | |
| wasser- und/oder öllösliche Farbstoffe | 0,05 | | | | |
| Füllstoffe/ Additive (Distärke-phosphat, $SiO_2$, BHT, Talkum, Aluminiumstearat) | 0,1 | 1 | 0,2 | 0,5 | 0,05 |
| Parfüm | q.s. | q.s. | q.s. | q.s. | q.s. |
| Wasser | Ad 100 | Ad 100 | Ad 100 | Ad 100 | Ad 100 |
| Glycerylsterat, selbstemulgierend | 2,5 | | | | 1 |
| PEG-40-Stearat | 1 | | | | |
| Polyglyceryl-3-Methylglucosedistearat | | 3 | | | |
| Sorbitanstearat | | 1 | | | |
| Polyethylenglycol(21)stearyl-ether (Steareth-21) | | | 2 | | |
| Polyethylenglycol(2)stearyl-ether (Steareth-2) | | | 1 | | |

(fortgesetzt)

| Beispiel Nummer | 1 | 2 | 3 | 4 | 5 |
|---|---|---|---|---|---|
| Cetearylglucosid | | | | 2 | |
| Stearinsäure | | | | | 2,5 |
| Myristylmyristat | | | | 1 | |
| Behenylalkohol | | 2 | | | 2 |
| Stearylalkohol | | | | 4 | |
| Cetearylalkohol | 2 | | 2 | | |
| Cetylalkohol | | 1 | | | 3 |
| Hydrierte Kokosfettglyceride (Hydrogenated Coco Glycerides) | 1 | | | | 1 |
| Sheabutter | 2 | | 1 | | |
| Macadamiaöl | | 1 | | | |
| C12-15 Alkylbenzoat | 4 | | 5 | 2 | 2 |
| Butylenglycol Dicaprylat/Dicaprat | | 2 | | | |
| Caprylsäure/Caprinsäure Triglycerid | 1 | 1 | | 3 | |
| Hydriertes Polydecen | | 1 | | 1 | |
| Octyldodecanol | 1 | | 1 | | 2 |
| Mineralöl | | | 1 | | |
| Iminodisuccinat | 0,2 | 0,2 | | | 0,1 |
| Taurin | 0,5 | 1 | 1 | 2 | 0,3 |
| p-Hydroxybenzoesäurebuthyllester | 0,1 | 0,05 | | 0,05 | |
| p-Hydroxybenzoesäuremethyllester | | 0,2 | 0,2 | | |
| Sorbinsäure | | | 0,2 | | |
| Iodopropynylbutylcarbamat | 0,05 | | | | 0,05 |
| Ethanol denaturiert | | 5 | | | 3 |
| 2-Ethylhexylglycerinether (Octoxyglycerin) | | | | 0,4 | |
| Xanthan Gummi | 0,1 | 0,2 | | 0,1 | |
| Polyacrylsäure (Carbomer) | | | 0,1 | | 0,2 |
| Ammoniumacryloyldimethyltaurate/ Vinylpyrrolidoncopolymere | | 0,3 | 0,3 | | |
| Aluminium Stärke Octenylsuccinate | | | | 0,5 | |
| Glycerin | 9 | 5 | 6 | 4 | 7 |
| Butylenglycol | | 5 | | 2 | |
| wasser- und/oder öllösliche Farbstoffe | | | | | 0,1 |
| Additive (Distärkephosphat, $SiO_2$, Talkum, BHT, Aluminiumstearat, $\gamma$-Tocopherol) | 0.03 | 0,1 | 0,05 | 3 | 1 |
| Parfüm | q.s. | q.s. | q.s. | q.s. | q.s. |
| Wasser | Ad 100 | Ad 100 | Ad 100 | Ad 100 | Ad 100 |

**Beispiel 11: W/O-Creme**

| | |
|---|---|
| Polyglyceryl-3-Diisostearat | 5,0 |
| Polyglyceryl-2-Dipolyhydroxystearat | 2,5 |
| Cetearylalkohol | 2 |
| Cetylalkohol | 2 |
| C12-15 Alkylbenzoat | 8 |
| Caprylsäure/Caprinsäure Triglycerid | 6 |
| Octyldodecanol | 5 |
| Octamethyltetrasiloxan (Cyclomethicon) | 2 |
| Milchsäure | 5 |
| Citronensäure, Natriumsalz | 0,5 |
| Butylmethoxydibenzoylmethan | 0,5 |
| Ethylhexyltriazon | 1 |
| Ethylhexylmethoxycinnamat | 5 |
| Taurin | 1 |
| Sorbinsäure | 0,2 |
| Retinylpalmitat | 0,05 |
| Phenoxyethanol | 0,1 |
| p-Hydroxybenzoesäurepropylester | 0,1 |
| Glycerin | 7.5 |
| Füllstoffe (Distärkephosphat, $SiO_2$, Talkum, Aluminiumstearat, BHT) | 0,2 |
| Iodopropynylbutylcarbamat | 0,05 |
| Wasser | Ad 100 |

**Beispiel 12: Hydrodispersion/Gelcreme**

| | |
|---|---|
| Cetylalkohol | 2 |
| Sheabutter | 1 |
| Caprylsäure/Caprinsäure Triglycerid | 2 |
| Octyldodecanol | 1 |
| Octamethyltetrasiloxan (Cyclomethicon) | 5 |
| Dimethylpolysiloxan (Dimethicon) | 1 |
| Polydecen | 2 |
| Ethylhexylmethoxycinnamat | 3 |
| Bis-Ethylhexyloxyphenol-methoxyphenyltriazin | 0,5 |
| Taurin | 0,5 |
| Natriumascorbylphosphat | 0,05 |
| Iodopropynylbutylcarbamat | 0,05 |

(fortgesetzt)

| | |
|---|---|
| Phenoxyethanol | 0,3 |
| p-Hydroxybenzoesäureethylester | 0,2 |
| Vernetztes Alkylacrylat (Alkylacrylate Cosspolymer) | 0,2 |
| Glycerin | 5 |
| Parfüm | q.s. |
| Wasser | Ad 100 |

**Beispielrezepturen Mikroemulsionen**

| | **1** | **2** | **3** | **4** |
|---|---|---|---|---|
| Lecithin | 1,8 | 3,0 | 1,0 | |
| PEG-50 Hydrogenated Castor Oil Isostearat | 5,2 | | | |
| PEG-20 Sorbitan Isostearat | | 4,0 | | 4,5 |
| Oleth-15 | | | 5,0 | |
| Glycerin | 5,0 | 5,0 | | |
| Dicaprylyl Ether | 7,0 | 7,0 | | |
| Phenoxyethanol | 0,5 | 0,4 | 0,5 | |
| p-Hydroxybenzoesäurepropylester | | | | 0,4 |
| Hexamidindiisethionat | | | 0,1 | |
| Diazolidinylharnstoff | 0,2 | 0,2 | | 0,1 |
| Iodopropynylbutylcarbamat | | | 0,1 | |
| Ethanol denaturiert | | 5 | | |
| 2-Ethylhexylglycerinether (Octoxyglycerin) | | | | 0,4 |
| Xanthan Gummi | | | 0,1 | |
| Tricontayl PVP | 0,3 | | | |
| Polyurethan-4 (Avalure UR-445) | | | 0,5 | |
| Taurin | 1 | 2 | 0,5 | 0,1 |
| Sorbinsäure | 0,05 | 0,2 | | 0,1 |
| Aluminium Stärke Octenylsuccinate | | | | 1 |
| Glycerin | 10 | 5 | 6 | 4 |
| Butylenglycol | | | | 2 |
| Wasser- und/oder öllösliche Farbstoffe | | 1 | | |
| Additive (Distärkephosphat, $SiO_2$, Talkum, BHT, Aluminiumstearat) | 0.03 | 0,1 | 0,05 | 3 |
| Parfüm | q.s. | q.s. | q.s. | q.s. |
| Wasser | ad 100 | ad 100 | ad 100 | ad 100 |

**Beispiele Pickering**

| | 1 | 2 | 3 | 4 |
|---|---|---|---|---|
| Mikrokristallines Wachs | 4,5 | 4 | 2,6 | 1,5 |
| Ozokerit | | 0,5 | 0,5 | 0,5 |
| Carnaubawachs | 1,5 | 2,1 | 1,8 | 1,8 |
| Candelillawachs | 4,0 | 2,0 | 2,0 | 6,0 |
| Bienenwachs | | 1,0 | | |
| C24-40 Alkyl Stearat | | | 2,0 | 1,5 |
| Lauryl Pyrrolidoncarbonsäure | | 3,0 | | |
| Lanolinöl | 4,0 | | | 2,0 |
| Bis-Diglyceryl Polyacyladipat-2 | 3,5 | 5,0 | | |
| Simethicon | 1,0 | | 0,5 | |
| Isopropyl Palmitat | 3,5 | 4,0 | | 2,0 |
| Triisostearin | 3,0 | | | |
| Myristyl Lactat | 4,0 | | 2,0 | 4,0 |
| Jojobaöl | 2,0 | | | 3,0 |
| Diisostearyl Malat | | | | 1,2 |
| Caprylic/Capric Triglycerid | | 5,0 | 3,0 | |
| Pentaerythrityl Tetraisostearat | | | 2,0 | |
| Isodecyl Neopentanoat | | 2,0 | | |
| Dicaprylyl Carbonat | | 2,0 | 2,0 | 3,0 |
| C12-15 Alkyl Benzoat | | | | 8,0 |
| Hydrogeniertes Polydecen | 2,5 | | | |
| Squalan | | | 2,0 | |
| Octyldodecanol | 2,5 | | ad 100 | 3,0 |
| Diisostearyl Fumarat | | | | ad 100 |
| PVP/Hexadecen Copolymer | | 0,5 | | |
| Taurin | 01 | 2 | 0,5 | 0,3 |
| Iodopropynylbutylcarbamat | 0,1 | 0,05 | | 0,1 |
| p-Hydroxybenzoesäuremethylester | | 0,1 | 0,2 | 0,3 |
| Ethylhexyl Methoxycinnamat | | 0,5 | | 2,0 |
| Butyl Methoxydibenzoylmethan | | 0,2 | | |
| Bis-Ethylhexyloxyphenol Methoxyphenyl Triazin | | | | 0,25 |
| Mikronisiertes Titandioxid (Eusolex T 2000) | 2,0 | 2,0 | 3,0 | |
| Silica Dimethyl Silylat (Aerosil R972) | | 0,3 | | |
| Polymethylsilsesquioxan (Tospearl 2000B) | | | 1,0 | 3,0 |
| Interferenz Pigmente | | 4,0 | 3,0 | |
| Titandioxid Cl 77891 | 4,0 | 3,8 | 2,0 | 3,0 |

(fortgesetzt)

| | 1 | 2 | 3 | 4 |
|---|---|---|---|---|
| Eisenoxide Cl 77491, 77492, 77499 | 3,2 | 2,2 | | 2,2 |
| D&C Rot 7 | 0,6 | | 1,9 | 1,3 |
| D&C Rot 6 | | | 0,4 | |
| FD&C Blau 1 | | | | 0,3 |
| D&C Rot 33 | | 0,3 | 2,2 | |
| D&C Rot 21 | | | | 0,5 |
| FD&C Gelb 6 | | 1,5 | | |
| D&C Rot 34 | | | 0,3 | 0,8 |
| Tocopheryl Acetat | 1,0 | | 1,5 | |
| Ubichinon | | | 0,1 | |
| Xylitol | 2,0 | | 2,0 | 2,0 |
| Glycerin | 5,0 | 3,0 | 3,0 | 5,0 |
| BHT, Parfum, Aroma | q.s. | q.s. | q.s. | q.s. |
| Wasser | 30,0 | 40,0 | 50,0 | 20,0 |
| Ricinusöl | ad 100 | ad 100 | 5,0 | |

**Beispiele W/O Stifte**

| | Pflegestift | Abdeckstift | Foundation Stift | Sonneschutzstift | Blushstift |
|---|---|---|---|---|---|
| Caprylic/Capric Triglyceride | 8 | 5 | 5 | 8 | 3 |
| Octyldodecanol | 7 | 5 | 5 | 8 | 6 |
| Carpylyl Carbonate | | | 3 | | |
| Dicaprylylether | | | 2 | | |
| Paraffinöl | 2 | | | | 1 |
| Pentaerythrityl Tetraisostearate | 2 4 | | | 8 | |
| C12-15 Alkyl Benzoate | 2 | | | | |
| Isopopyl Palmitate | | | | | 2 |
| Jojobaöl | 2 | | | 1 | 1 |
| Lanolinöl | | | | | 1 |
| Simethicone | | 0,5 | 0,5 | | 0,5 |
| PEG-45/ Dodecyl Glycol Copolymer | 3 | 3,5 | 2 | | 2 |
| Polyglyceryl-3 Diisostearate | 2,5 | | 1,5 | 2 | 2,4 |
| PEG-30 Di-Polyhydroxystearate | | | | 2,5 | |
| Sucrose Distearate | 0,5 | | | | |
| Bis-Diglyceryl Polyacyladipate-2 | 9 | 2 | | | |
| Cetyl Palmitate | 2,5 | | | | 1 |
| C16-36 Alkyl Stearate | 14 | 1 | 2 | 1 | |
| C20-40 Alkyl Stearate | | 8 8 | | 9 8 | |
| Carnaubawachs | 1,5 | 1,5 | | | 2 |
| Bienenwachs | 0,5 | | | | |
| Candelillawachs | | | | | 1 |
| PVP / Eicosene Copolymer | | 1 | | 1 | 0,2 |
| Butyl Methoxydibenzoylmethane | | | | 1 | |
| Mikronisiertes Titan Dioxid | | 2 | | 4 | |

(fortgesetzt)

| | **Pflegestift** | **Abdeckstift** | **Foundation Stift** | **Sonneschutzstift** | **Blushstift** |
|---|---|---|---|---|---|
| Ethylhexylcyanodiphenyl-acrylat (Octocrylen) | 2 | | | 3,6 | 5 |
| Octyl Methoxycinnamate | | 2 | | 3,6 | 2,5 |
| Taurin | 1 | 0,2 | 0,7 | 0,1 | 2 |
| Phenoxyethanol | 0,4 | 0,4 | 0,2 | 0,1 | |
| Iodopropynylbutylcarbamat | 0 | 0,1 | 0,05 | | 0,05 |
| p-Hydroxybenzoesäuremethylester | 0,3 | 0,1 | | 0,2 | 0,3 |
| Bismuthoxichlorid (BiOCl) | 2 3 | | | | 2 |
| Bornitrid | | | | 3 | 1 |
| Lauroyl Lysine | | 0,5 | | | |
| Polymethylsilsesquioxane (Tospearl) | | | 0,5 | 1 | |
| Silica LDP | | | | 1 | |
| PTFE | 2,5 | | | | |
| PMMA | | 6 | 3 | | |
| Titandioxid $Al_2O_3$ gecoated | | 7 6 | | | 2 |
| Eisenoxide | | 4 4 | | | 6 |
| Ultramarin | | 0,5 | 0,6 | | |
| Perlglanzpigmente | 3 | | | | 2 |
| Rokonsal S1 | 0,4 | | | | |
| Germall II | | 0,25 | | | 0,25 |
| Glydant Plus | | | | 0,3 | |
| Glycerin | 5 2 | | 10 | 5 | 10 |
| Parfüm, BHT, Neutralisationsmittel, | q.s | q.s | q.s | q.s | q.s |
| Wasser | ad 100 | ad 100 | ad 100 | ad 100 | ad 100 |

## Patentansprüche

1. Kosmetische oder dermatologische Zubereitungen enthaltend Taurin und einen Stoff gewählt aus der Gruppe Parabene, Sorbinsäure oder deren Alkalisalze, IPBC.

2. Zubereitung nach einem der vorangehenden Ansprüche **dadurch gekennzeichnet, daß** der Stoff gewählt aus der Gruppe Parabene, Sorbinsäure, IPBC in Konzentrationen von 0.001 bis 1 Gew.%, besonders bevorzugt 0,01 bis 0,5 Gew.% vorliegt.

3. Zubereitung nach einem der vorangehenden Ansprüche **dadurch gekennzeichnet, daß** Taurin in Konzentrationen von 0,001 bis 5 Gew.%, besonders bevorzugt 0.01-0.5 Gew.% vorliegt.

4. Zubereitung nach einem der vorangehenden Ansprüche **dadurch gekennzeichnet, daß** das Verhältnis von Taurin zur Gesamtmenge an Stoffen gewählt aus der Gruppe Parabene, Sorbinsäure, IPBC 1:100 bis 1000:1, besonders bevorzugt 5:1 bis 1:5 beträgt.

5. Zubereitung nach einem der vorangehenden Ansprüche **dadurch gekennzeichnet, dass** sie in Form einer O/W-Emulsion oder Hydrodispersion vorliegt.